# EUROPEAN PATENT APPLICATION

(11) **EP 4 001 295 A1**
(43) Date of publication of application: **25.05.2022**
(21) Application number: 20841421.9
(22) Date of filing: 30.06.2020
(51) Int. Cl.: C07K 7/08, A61K 47/64

(54) **PEPTIDE THERAPEUTICS FOR AUTOIMMUNE DISEASES AND INFLAMMATORY DISEASES**

(30) Priority: 16.07.2019 KR 20190085927
(71) Applicant: Genesen Co., Ltd., Seoul 05836 (KR)
(72) Inventor: CHOI, Sang Dun, Suwon-si Gyeonggi-do 16493 (KR); SUH, Chang Hee, Seoul 06288 (KR); KIM, Wook, Suwon-si Gyeonggi-do 16509 (KR); SHAH, Masaud, Suwon-si Gyeonggi-do 16499 (KR); ACHEK, Asma, Suwon-si Gyeonggi-do 16499 (KR); KIM, Giyoung, Incheon 21980 (KR); CHEONG, Jae Youn, Suwon-si Gyeonggi-do 16499 (KR); KIM, Soon Sun, Suwon-si Gyeonggi-do 16499 (KR)
(74) Representative: Dehns
(86) International application number: PCT/KR2020/008536
(87) International publication number: WO 2021/010621

(57) **Abstract**

The present invention relates to a peptide inhibiting a Toll-like receptor (TLR) signaling pathway. More specifically, the present invention relates to: a peptide that strongly binds to a TIR-containing molecule to inhibit the TLR, in particular, the TLR4 signaling pathway; a fusion peptide in which a cell-penetrating peptide is conjugated to said peptide; a TLR4 antagonist comprising said peptide or said fusion peptide; and a composition for preventing or treating autoimmune diseases or inflammatory diseases comprising said peptide or said fusion peptide.

According to the present invention, said peptide exhibits an inhibitory effect on a wide range of TLR signaling pathways including TLR4, blocks MyD88- and TRIF-dependent TLR4 pathways, and has a substantial disease-alleviating effect in mouse models of rheumatoid arthritis, psoriasis, systemic lupus erythematosus, and non-alcoholic steatohepatitis, and thus can be usefully utilized as therapeutics for immune-related diseases and inflammatory diseases that require negative control of TLR.

<110>    AJOU UNIVERSITY INDUSTRY-ACADEMIC COOPERATION FOUNDATION

<120>    Peptide Therapeutic Agent of Autoimmune Diseases and Inflammatory
         Diseases

<130>    PP-B2418

<150>    KR 2019-0085927
<151>    2019-07-16

<160>    8

<170>    KoPatentIn 3.0

<210>    1
<211>    12
<212>    PRT
<213>    Artificial Sequence

<220>
<223>    MIP


<400>    1
Pro Trp Cys Lys Tyr Gln Met Leu Gln Ala Leu Thr
  1               5                   10


<210>    2
<211>    12
<212>    PRT
<213>    Artificial Sequence

<220>
<223>    MIP


<400>    2
Pro Trp Ala Lys Tyr Gln Ala Leu Gln Ala His Thr
  1               5                   10


<210>    3
<211>    12
<212>    PRT
<213>    Artificial Sequence

<220>
<223>    MIP


<400>    3
Pro Trp Ala Lys Tyr Gln Met Leu Gln Ala Leu Thr
  1               5                   10


<210>    4
<211>    6
<212>    PRT
<213>    Artificial Sequence

<220>
<223>    MIP

<400>    4
Pro Trp Cys Lys Tyr Gln
1               5

<210>    5
<211>    12
<212>    PRT
<213>    Artificial Sequence

<220>
<223>    MIP

<400>    5
Pro Trp Cys Lys Tyr Gln Cys Leu Gln Ala Leu Thr
1               5                   10

<210>    6
<211>    16
<212>    PRT
<213>    Artificial Sequence

<220>
<223>    cell penetrating peptide

<400>    6
Arg Gln Ile Lys Ile Trp Phe Gln Asn Arg Arg Met Lys Trp Lys Lys
1               5                   10                  15

<210>    7
<211>    6
<212>    PRT
<213>    Artificial Sequence

<220>
<223>    cell penetrating peptide

<400>    7
Pro Phe Val Tyr Leu Ile
1               5

<210>    8
<211>    28
<212>    PRT
<213>    Artificial Sequence

<220>
<223>    CPP-MIP2

<400>    8
Arg Gln Ile Lys Ile Trp Phe Gln Asn Arg Arg Met Lys Trp Lys Lys
1               5                   10                  15

Pro Trp Cys Lys Tyr Gln Met Leu Gln Ala Leu Thr
            20                  25

## Description

### Technical Field

The present invention relates to a peptide that inhibits signaling pathways of a Toll-like receptor (TLR), and more specifically, to a peptide that strongly binds to a TIR-containing molecule and thereby inhibits the signaling pathway of TLR (in particular TLR4), a fusion peptide in which a cell penetrating peptide is bound to the peptide, an TLR antagonist comprising the peptide or fusion peptide, and a pharmaceutical composition for preventing or treating a TLR pathway-mediated disease, comprising the same.

### Background Art

Signaling of a Toll-like receptor (TLR), which belongs to a family of pattern recognition receptors, is essential for pathogen recognition by the innate immune system. TLRs recognize unique properties presented by pathogens or by dying/damaged cells during infection. These characteristics are known as danger-associated molecular patterns (DAMPs) and pathogen-associated molecular patterns (PAMPs) (Akira, S., Uematsu, S. & Takeuchi, O. Cell 124, 783-801 (2006)). Upon recognition of DAMPs/PAMPs, the Toll/interleukin-1 receptor (TIR) domain of TLR self-associates, which is essential for signaling. Subsequently, recruitment of other TIR-containing adapter molecules, including TRIF (TICAM-1), MAL (TIRAP), MyD88, and TRAM (TICAM-2), and activation of NF-κB, interferon regulators (IRFs), and other transcription factors are followed (Gay, N. J., et al., Nat Rev Immunol 14, 546-558 (2014)). The recruitment mechanism of adapter proteins is not similar in all TLRs. TLRs that signal through MyD88 may be MAL-dependent or they may bind directly to the TLR-TIR domain to induce a downstream signaling cascade. Meanwhile, TLR3 regulates signaling through TRIF by MyD88 and TRAM themselves.

Once activated, TLRs aggregate to lipid rafts located in the plasma membrane and initiate downstream signaling in a highly sophisticated manner. The precise alignment of TLR-TIRs and adapter molecules is important for understanding how TLRs transduce signals. In order to decipher TIR-TIR molecular interactions, many empirical models (Chen, CJ et al., Nat Med 13, 851-856 (2007)) and *in silico* models (Guven-Maiorov, E. et al., Sci Rep 5 , 13128 (2015)) have been proposed. Nevertheless, due to the complexity, the entire signalosome structure has not yet been elucidated. Molecular docking, mutagenesis, and crystal contact analysis have been used to propose stoichiometric oligomerization of the TIR-TIR model and elucidate their interaction interfaces, but still no general TIR-TIR interface has been observed for mammalian TIR domains. Eventually, a stoichiometric relationship of TIR-TIR interactions in the signalosome has been hypothesized, and the mechanisms underlying this interaction-cascade are vaguely understood.

Among the TIR-domain-containing adapter proteins, MAL (a TIR domain adapter protein; TIRAP) has the ability to link Myeloid differentiation primary response 88 (MyD88) and TLR2 or TLR4. An N-terminus containing a positively charged motif anchors MAL to the plasma membrane via a PIP2 molecule (Patra, M. C. & Choi, S., Front Immunol 9, 75 (2018)). MAL-TIR can be present in the form of a back to back symmetric dimer promoted by the αC and αD helices, and as a CD loop. In contrast, recent cryo-electron microscopy (cryo-EM) studies have suggested that MAL-TIR forms spontaneous and reversible filaments promoted by a BB-loop *in vitro.* This is probably because the previous crystal structure could not capture the other flexible regions responsible for the BB-loop and TIR-TIR intrastrand interactions. According to the cryo-EM structure, MAL forms an intra-chain interaction by the BB-loop in a head-to-tail manner, and it forms an interstrand-interaction including the αB and αC helices and αD helices of one MAL-TIR and the CD loop of the other MAL-TIR. Thus, the interchain-interactions are maintained in a triangular fashion. That is, one subunit interacts with two other subunits in the protofilament. The interactions between these triangular chains involving the αC helices are similar to the dimer symmetric interface proposed by Lin *et al.* The MAL-TIR-filament also induces the MyD88 assembly and forms a co-filament with the TLR4-TIR.

Disruption of interchain interactions by mutating the W156, K158, Y159, L162 and L165 residues in the αC helix completely destroys the filament formation in MAL-TIR. It has also been reported that residues present in the αC helix interfere with the MAL-MAL interaction. This was confirmed through a mammalian protein-protein interaction trap (MAPPIT) method. The W156A mutation in αC substantially affected the MAL-MyD88 interaction (Bovijn, C. et al., J Biol Chem 288, 12054-12066 (2013)). Mutations at the symmetric interface could also inhibit the interaction of MAL-TLR4 intracellular (MAL-TLR4ic) domain in MAPPIT. Furthermore, in a manner similar to the MAL-MAL interchain-interface, a plausible interchain-interface for the TLR4-TLR4-MAL TIR interaction has been proposed. These results suggest a crucial role for MAL-αC in the TIR-TIR interaction and promotion of downstream signaling cascades.

Considering the important role of MAL in TLR2 and TLR4 signaling, MAL-derived decoy peptides have been designed, and TLR4 and TLR2 antagonistic capacities have been reported (Couture, L. A., et al., J Biol Chem 287, 24641-24648 (2012)). However, it was suggested that peptides derived from the N-terminal half of αC together with the CC loop (TR6) can inhibit TLR2/1 and TLR4 signaling, but not the C-terminal half together with the CD loop (TR7). In contrast, Lin *et al.* reported that Q163A and L165A (located at the C-terminus) mutations could reduce NF-κB induction by 60% and 20%, respectively. Structural studies by Thomas *et al.* further support the role of L165 in MAL protofilament assembly (Lin, Z., et al., PLoS One 7, e34202 (2012)).

Therefore, the present inventors have hypothesized that the C-terminus of αC would be as important as half of the N-terminus and that, due to the stable helical structure, a peptide containing the amino acids P155-T166 would be sufficient to destroy the MAL-TIR assembly as well as other MyD88-dependent TLR signaling.

The present inventors have made extensive efforts to develop a new decoy peptide capable of inhibiting a wide range of TLR signaling pathways, and as a result, they have designed a peptide derived from the αC helix of MAL (defined as "MIP2") and have confirmed that the peptide bound to a cell penetrating peptide successfully inhibited TLR signaling, and have confirmed that it alleviates the symptoms of psoriasis in mice, alleviates the symptoms observed in a systemic lupus erythematosus mouse model, and shows therapeutic effects in a mouse model of arthritis, thereby completing the present invention.

The information provided in the Background section above is merely for the purpose of improving the understanding of the background of the present invention. Therefore, the information constituting the prior art known to one of ordinary skill in the art to which the present invention pertains may not be included.

### Disclosure of Invention

### Technical Problem

An object of the present invention is to provide a peptide which inhibits a TLR signaling pathway, and a fusion peptide in which a cell penetrating peptide is bound to the above peptide.

Another object of the present invention is to provide a TLR antagonist which comprises the peptide or the fusion peptide.

Still another object of the present invention is to provide a composition for preventing or treating autoimmune diseases or inflammatory diseases, comprising the peptide or the fusion peptide.

Still another object of the present invention is to provide a method for preventing or treating autoimmune diseases or inflammatory diseases, comprising a step of administering the peptide or the fusion peptide; a use of the peptide or the fusion peptide for preventing or treating autoimmune diseases or inflammatory diseases; and a use of the peptide or the fusion peptide in preparation of a medicament for preventing or treating autoimmune diseases or inflammatory diseases.

### Solution to Problem

In order to achieve the above objects, the present invention provides a peptide represented by the amino acid sequence of SEQ ID NO: 1.

The present invention also provides a fusion peptide in which a cell penetrating peptide is bound to the peptide.

The present invention also provides a TLR antagonist comprising the peptide or the fusion peptide.

The present invention also provides a composition for preventing or treating autoimmune diseases, comprising the peptide or the fusion peptide.

The present invention also provides a composition for preventing or treating inflammatory diseases, comprising the peptide or the fusion peptide.

The present invention also provides a method for preventing or treating autoimmune diseases or inflammatory diseases, comprising a step of administering the peptide or the fusion peptide.

The present invention also provides a use of the peptide or the fusion peptide for preventing or treating autoimmune diseases or inflammatory diseases.

The present invention also provides a use of the peptide or the fusion peptide in preparation of a medicament for preventing or treating autoimmune diseases or inflammatory diseases.

### Brief Description of Drawings

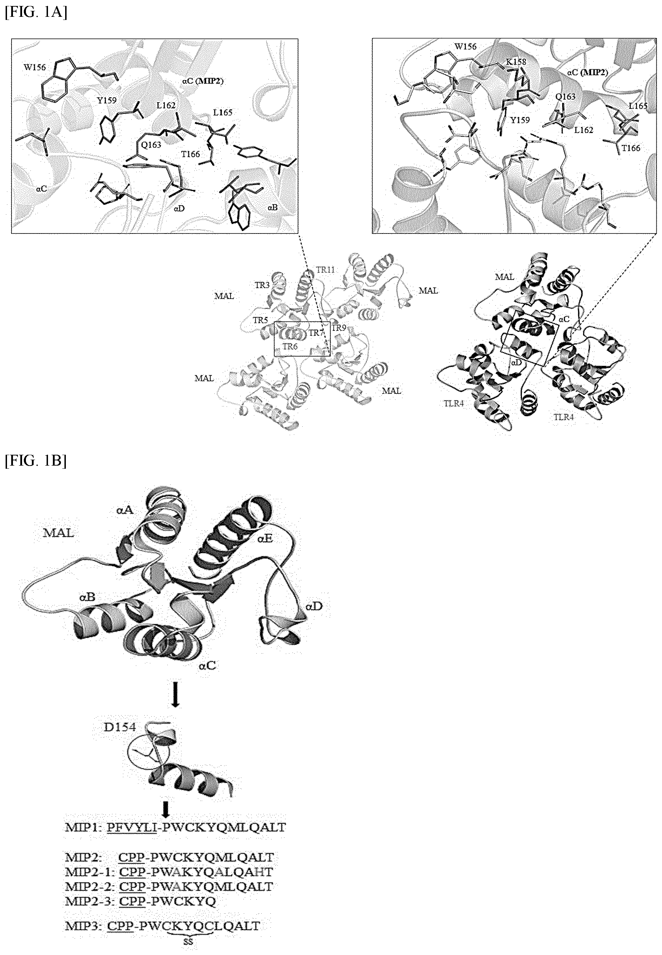

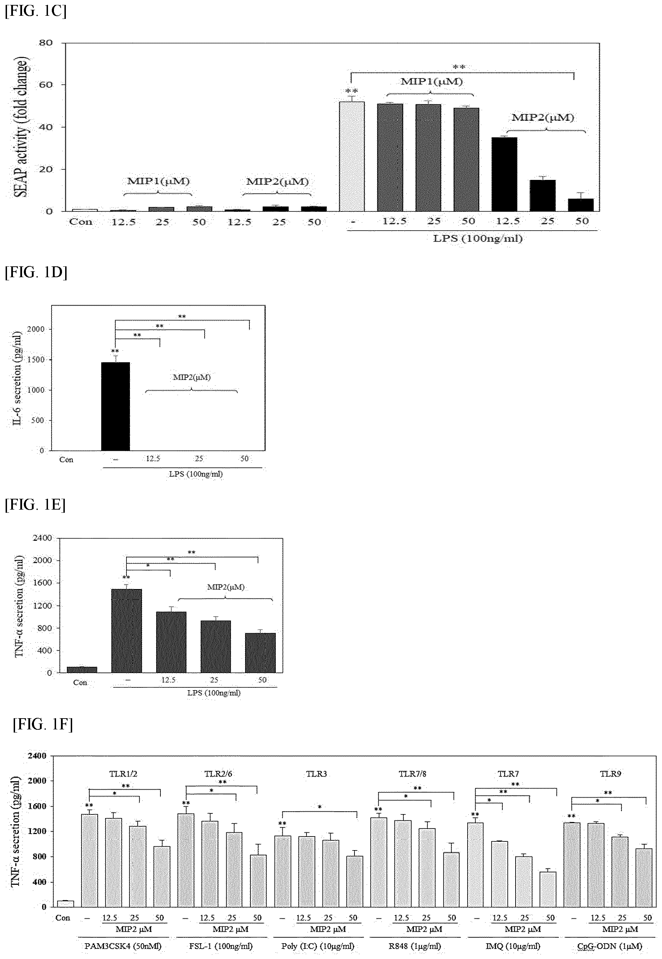

FIG. 1 shows peptide designs and *in vitro* screening, in which FIG. 1A shows MAL and the αC of MAL which promotes a TIR-TIR interaction with other TIR-containing adapter proteins; FIG. 1B shows the αC helices of MAL used to design the MAL/MyD88 inhibitory peptide (MIP) and a derivative thereof using a computer; FIG. 1C shows a graph illustrating the measurement results of the TLR-inhibitory effect of MIP1 and MIP2 by measuring the SEAP signal in HEK-Blue^{™} hTLR4 cells; FIGS. ID and 1E show graphs illustrating the secretion levels of IL-6 and TNF-α measured by ELISA in RAW264.7 cells; and FIG. 1F shows a graph illustrating the broad TLR-inhibitory effect of MIP2 evaluated by measuring TNF-α levels in RAW264.7 cells, in which the cells were activated by treating with various concentrations of PAM₃CSK₄ (TLR1/2), FSL-1 (TLR2/6), Poly(I:C) (TLR3), R848 (TLR7/8), Imiquimod (IMQ; TLR7), or CpG-ODN (TLR9) (the data shown were obtained from at least 3 independent experiments (n ≥ 3), each bar represents mean ± SEM (^{∗}P < 0.05, ^{∗∗}P < 0.01)).

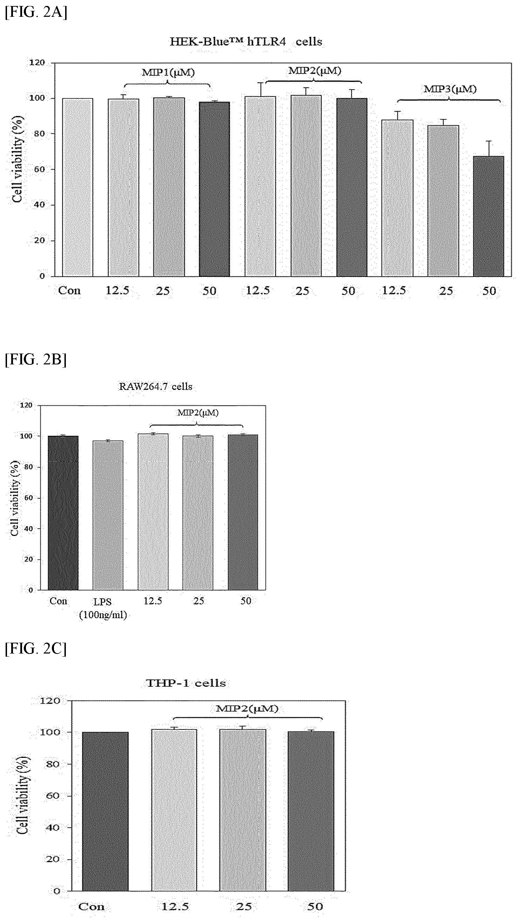

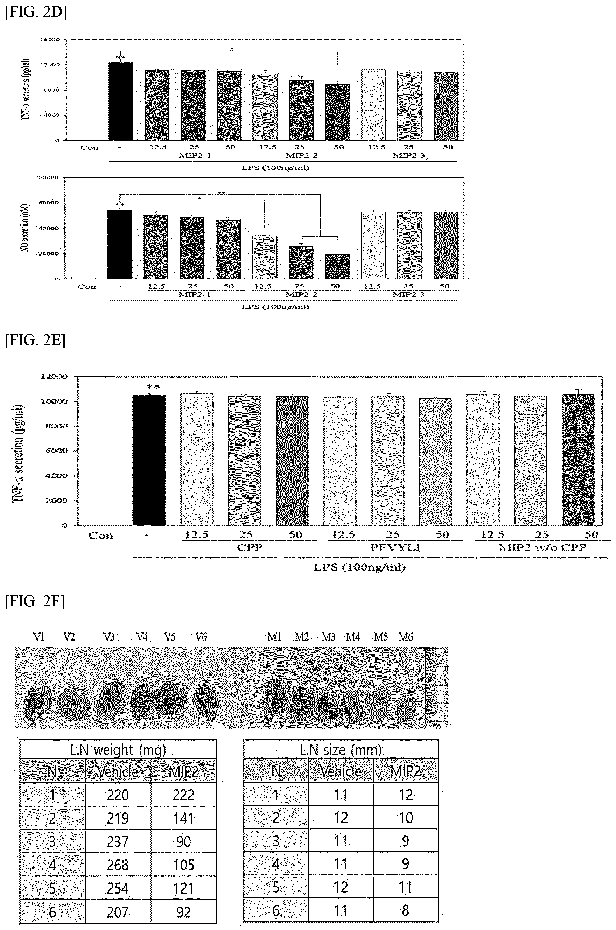

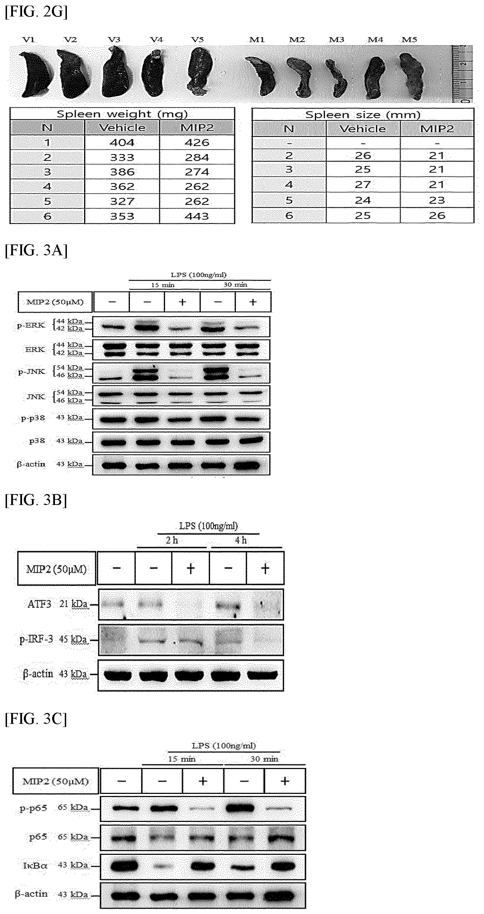

FIG. 2 shows evaluation results of the toxicity of each peptide named MIP1, 2, and 3 in HEK-Blue^{™} hTLR4, RAW264.7, and THP-1 cells, in which FIG. 2A shows that MIP1 and MIP2 peptides do not show toxicity in HEK-Blue^{™} hTLR4 cells; FIGS. 2B and 2C show graphs illustrating that MIP2 does not show toxicity even in RAW264.7 and THP-1 cells; FIG. 2D shows graphs illustrating that MIP2-1, MIP2-2, and MIP2-3 inhibit or do not inhibit the secretion of TNF-α cytokine and NO induced by LPS, respectively; FIG. 2E shows responses of MIP2 without CPP, CPP alone, and PFVYLI (which is a hydrophobic CPP) to LPS-stimulated RAW264.7 cells; and FIGS. 2F and 2G show the comparison results of the weight and size of lymph nodes and spleens of systemic lupus erythematosus mice after ablation.

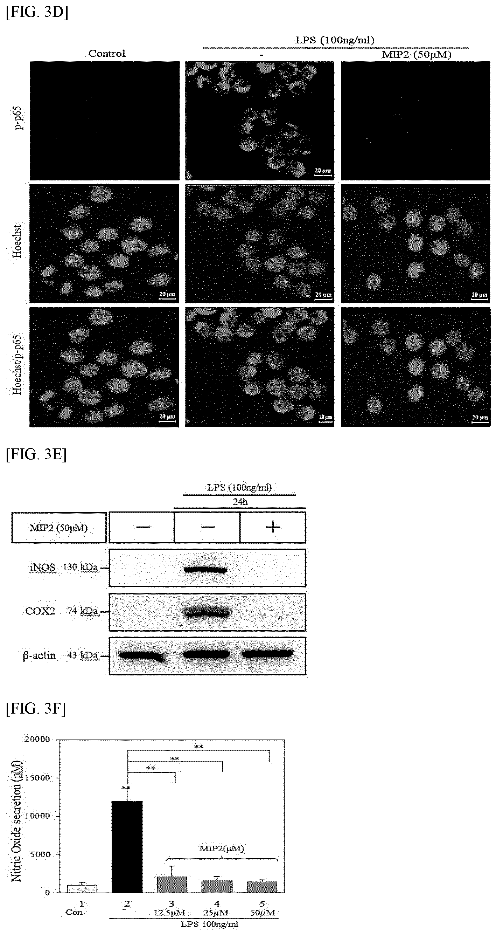

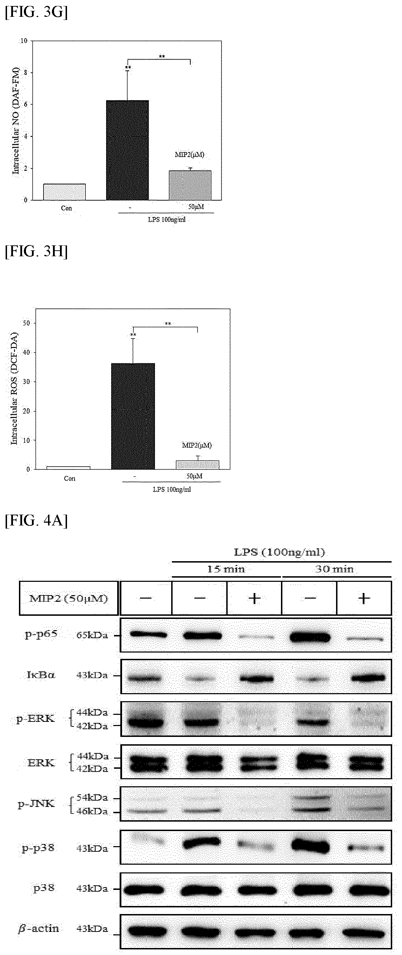

FIG. 3 shows MIP2 which inhibits MyD88- and TRIF-dependent TLR4 signaling in mouse macrophages, in which FIGS. 3A to 3C show the protein expression levels of p-p65, p65, Iκ-Bα, ATF3, p-IRF3, p-ERK, ERK, p-JNK, JNK, p-p38, and p38 in RAW264.7 cells determined by Western blot analysis of total-protein extraction, where β-actin was used as a loading control; FIG. 3D shows images of phosphorylation of NF-κB (p-p65) measured by immunofluorescence staining and confocal microscopy, where Hoechst was used for nuclear staining (the scale bar represents 20 µm); FIG. 3E shows the expression levels of iNOS and COX2, which were completely inhibited by MIP2, measured by Western blot, where β-actin was used as a loading control; FIG. 3F shows a graph illustrating the level of NO secretion measured with a standard NO secretion kit; and FIGS. 3G and 3H show graphs illustrating the production of intracellular NO and ROS, which were quantified by DAF-FM and DCF-DA staining, respectively, and substantially downregulated by MIP2 (the data shown were obtained from at least 3 independent experiments (n ≥ 3), each bar represents mean ± SEM (^{∗}P < 0.05, ^{∗∗}P < 0.01)).

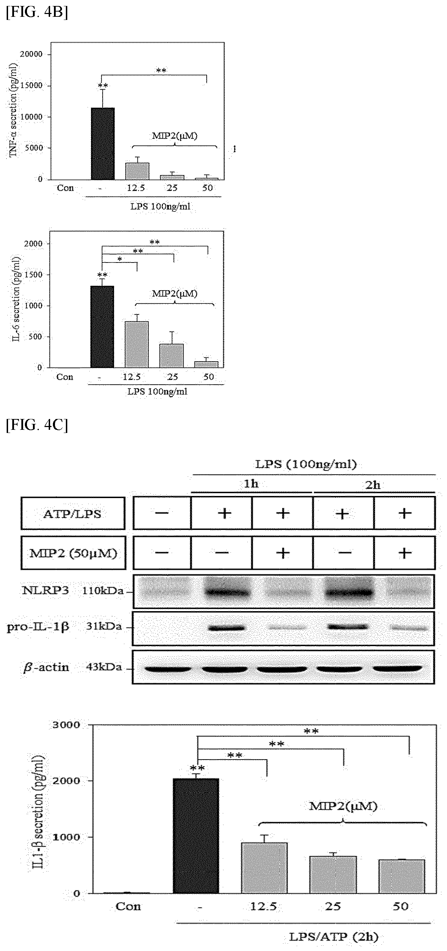

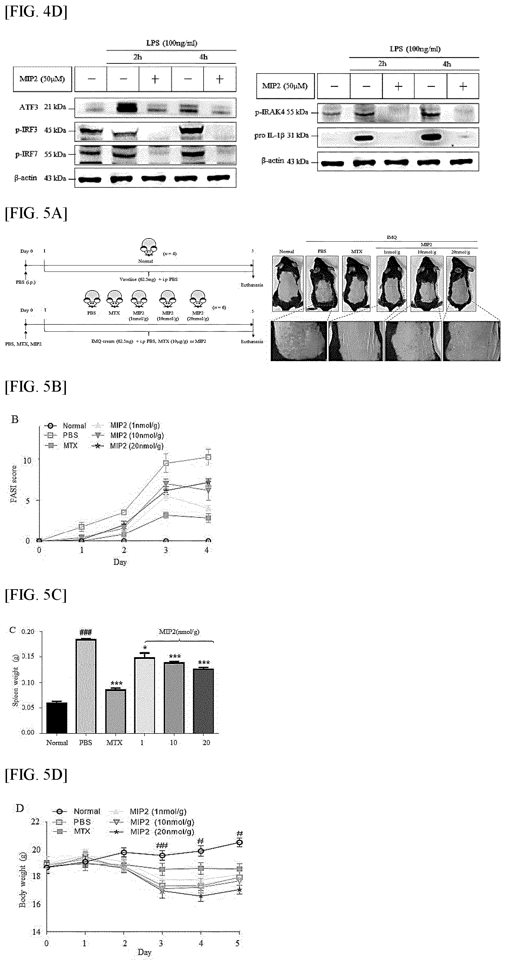

FIG. 4 shows MIP2 which inhibits the TLR and NLRP3 pathways in THP-1 cells, in which FIG. 4A shows the expression of multiple transcription factors including p-p65, Iκ-Bα, p-ERK, ERK, p-JNK, JNK, p-p38, and p38 measured by Western blot analysis, where β-actin was used as a loading control and the phosphorylated state of the factors was substantially inhibited by MIP2; FIG. 4B shows graphs illustrating that the secretion levels of TNF-α and IL-6 are inhibited in a concentration-dependent manner by MIP2 when evaluated by ELISA; FIG. 4C shows the inhibitory effect of MIP2 against NLPR3 measured in ATP-treated (10 mM) THP-1 cells after being primed by LPS (100 ng/mL for 4 hours), where the expression levels of NLRP3 and pro-IL1β were measured by Western blot, and cytokine secretion of IL1β was evaluated using ELISA; and FIG. 4D shows the expression levels of ATF3, p-IRF3, p-IRF7, pIRAK4, and pro-IL1β measured by Western blot analysis in LPS-stimulated cells, where β-actin was used as a loading control (the data shown were obtained from at least 3 independent experiments (n ≥ 3), each bar represents mean ± SEM (^{∗}P < 0.05, ^{∗∗}P < 0.01)).

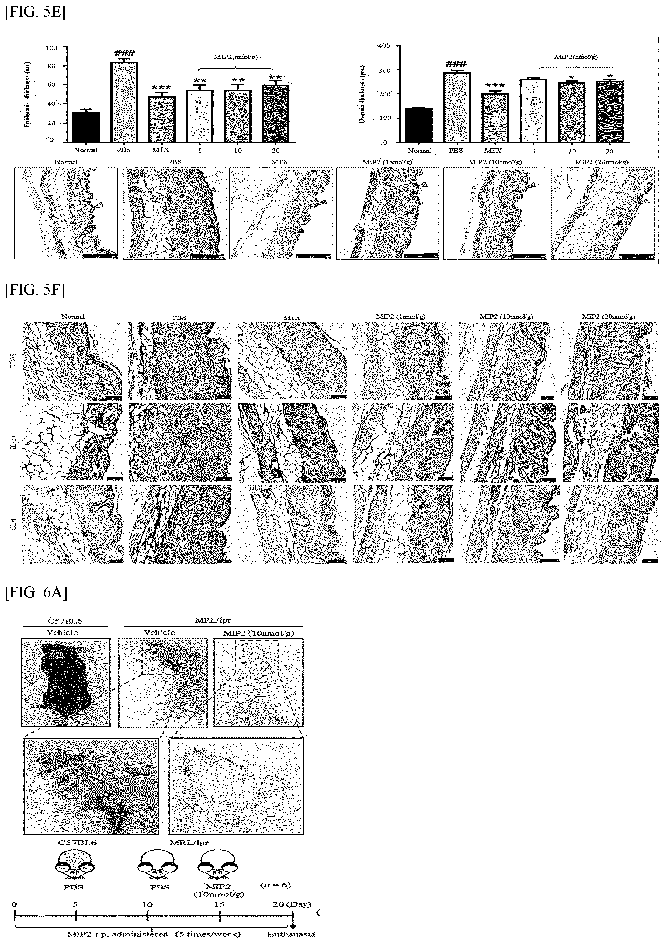

FIG. 5 shows MIP2 which improves imiquimod-induced psoriasis in mice, in which FIG. 5A shows psoriasis induced by topical application of imiquimod (IMQ) in C57BL/6 male mice, in which three concentrations of MIP2 were administered intraperitoneally before IMQ application, and methotrexate (MTX) was used as a positive control for the relative treatment evaluation of MIP2, the images of back skin were photographed on the 4th day of treatment, showing that MIP2 had a therapeutic effect compared to the normal, untreated, and MTX-treated groups; FIG. 5B shows a graph illustrating the disease severity score based on the Clinical Psoriasis Area and Severity Index (PASI), where the three purple lines represent the PASI scores of mice treated with 1 nmol/g, 10 nmol/g, and 20 nmol/g of MIP2, respectively; FIGS. 5C and 5D show graphs illustrating the effect of MIP2 on spleen weight and body weight kinetics of mice during treatment regimen; FIG. 5E shows results illustrating the effect of MIP2 on the thickness of the epidermis (grey arrows) and the dermis (black arrows), where the skin thickness of each group was measured with a Leica DMi8 fluorescence microscope (scale bar: 250 µm); and FIG. 5F shows the images illustrating the results of immunohistochemical analysis of dorsal skin lesions in each group, where the intensity of brown staining suggests CD68 (a macrophage marker) and CD4 and IL17 (TH17 cell markers) in the lesion, and the scale bar used is 75 µm for high magnification images (data represents mean ± SEM obtained from 5 skin tissue samples in each group) (###P < 0.001 between NC and PBS, ^{∗∗}P < 0.01, ^{∗}P < 0.05 between PBS and MIP2 at 1 nmol, 10 nmol, or 20 nmol or MTX, according to two-tailed Student's t-test).

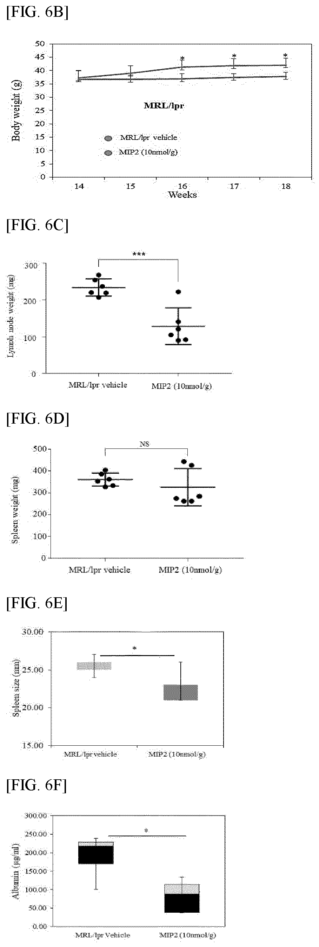

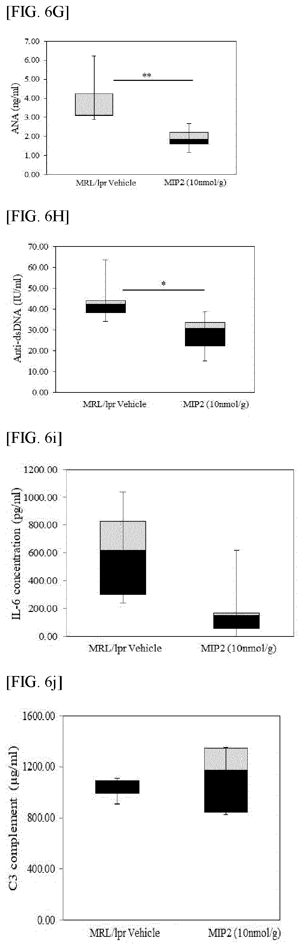

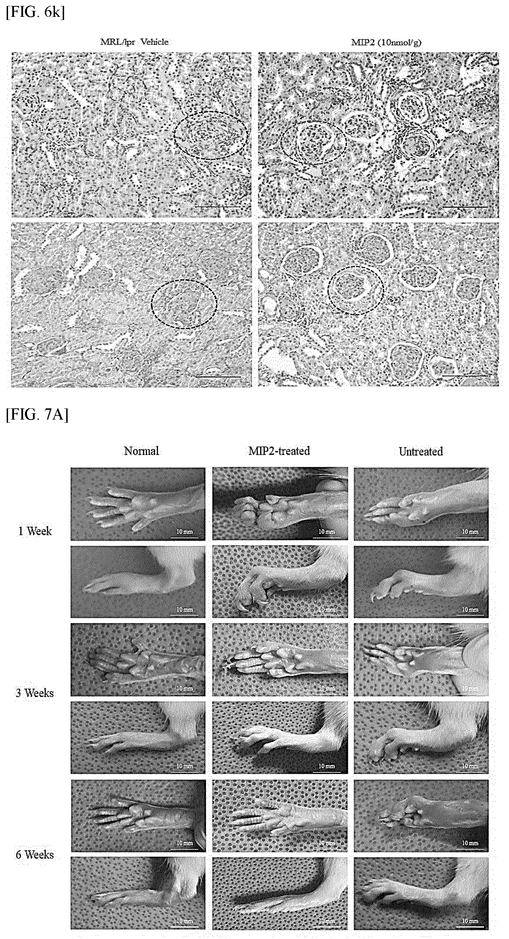

FIG. 6 shows the therapeutic effect of MIP2 in a SLE mouse model, in which FIG. 6A shows the experimental verification results of the inhibitory effect of MIP2 in a SLE mouse model, where a significant reduction in hair loss was observed in SLE-prone MIP2-treated mice; FIG. 6B shows a graph illustrating that body weight did not change in MIP-treated mice; FIGS. 6C to 6E show graphs illustrating that lymphadenopathy and splenomegaly are significantly improved in MIP2-treated SLE-prone mice; FIG. 6F shows the albumin content in the urine; FIGS. 6G to 6J show the results of evaluation of serum ANA, an anti-dsDNA antibody, IL-6, and C3 complement evaluated by ELISA; and FIG. 6K shows the result of histological examination of the kidney through H & E and PAS staining (scale bar: 100 µm), where a glomerular mass with proliferative mesangial cells and diffused black broken circles were found in untreated mice, whereas fibrocellular crescent (blue circle) was reformed and the glomeruli returned to normal in MIP2-treated mice.

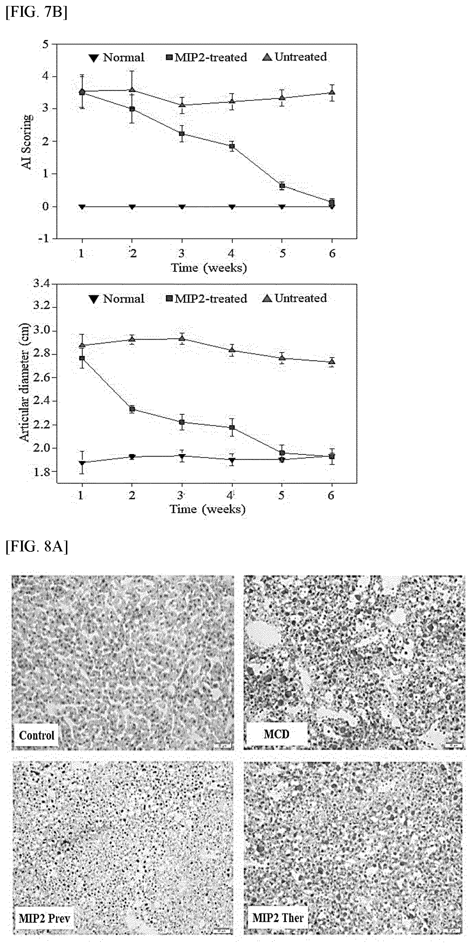

FIG. 7 shows the therapeutic effect of MIP2 in a rheumatoid arthritis (RA) rat model, in which FIG. 7A shows a paw monitoring result of normal, MIP2-treated (20 pmol/g of rat's weight), and untreated RA rats at weeks 1-6 (scale bar: 10 mm); FIG. 7B shows an accurate Wilcoxon Rank Sum test (numerically identical to the Mann-Whitney U test) was performed to compare the average values between the two groups with the index scores of ankle diameter and joints of rats measured at weeks 1-6 (^{∗}P < 0.05 and ^{∗∗}P < 0.01).

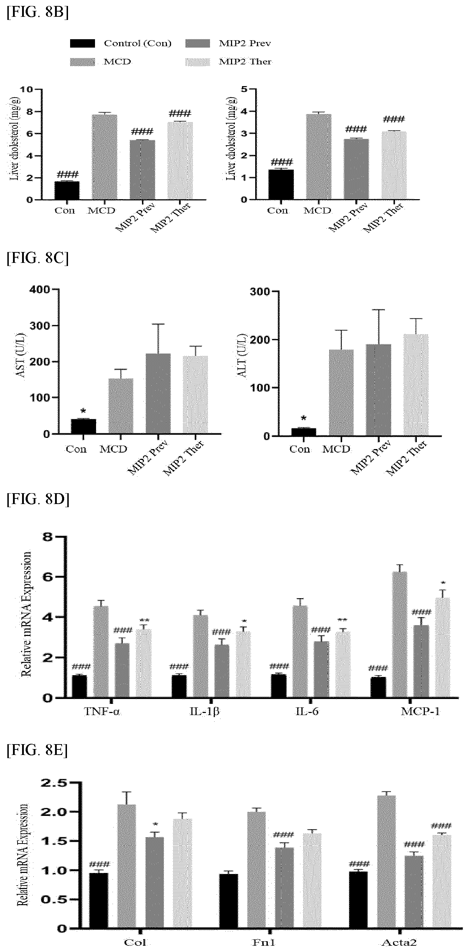

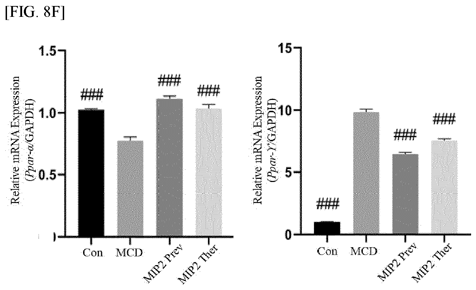

FIG. 8A shows the histological examination of the liver through Oil red O stain (scale bar: 50µm) where significant changes in macrovesicular fat were induced by the methionine-choline-deficient (MCD) diet, and MIP2 did not show any significant improvement in steatosis in a proventative and therapeutic manner (MIP2 Prev and MIP2 Ther, respectively); FIG. 8B shows cholesterol and triglyceride levels in the liver of a group of four mice, where it was confirmed that the changes in liver fat induced by MCD diet showed an improved in both MIP2 Prev and MIP2 Ther groups; serum AST and ALT levels were not significantly improved by MIP2 (FIG. 8C); inflammation (FIG. 8D) and fibrosis (FIG. 8E)-associated gene transcription factors were significantly improved with MIP2 administration; and dysregulated Pparα and Ppar transcription factor levels were restored with MIP2 administration (FIG. 8F).

### Best Mode for Carrying out the Invention

Unless defined otherwise, all technical and scientific terms used herein have the same meaning as commonly understood by one of ordinary skill in the art to which the present invention belongs. In general, the nomenclature used herein is those which are well known and commonly used in the art.

Toll-like receptors (TLRs) play an important role in the fight against microorganisms; however, they have been implicated in the exacerbation of several immune disorders (*e.g*., systemic lupus erythematosus (SLE), psoriasis, and rheumatoid arthritis (RA)) due to their dysregulated signaling. The stoichiometry and precise alignment of adapter TIR-TIR interactions are essential for TLR activation and the underlying signaling-cascade. Among the adapters, plasma membrane-anchored MALs have the potential for BB-loop-mediated self-oligomerization and interact with other TIR-domain containing adapters via αC and αD helices.

Among the TIR-domains that contain adapters, MAL can link MyD88 and TLR9. According to mutation studies, it was confirmed that the αC helix of MAL is important not only for MAL-MAL, but also for MAL-MyD88 and MAL-TLR4 interactions. Mutations in the C-terminal residues of the αC helix (Q163A and L165A) were reported to inhibit NF-κB induction by 60% and 20%, respectively. The interchain-interface of MAL-protofilament has been proposed to be similar to the MAL-MyD88 and MAL-TLR4/2 TIR-TIR interfaces. In the model, it is proposed that the αD helix of TLR4 forms a major contact with the αC helix of MAL (FIG. 1A). According to Tashchako *et al.,* peptides derived from the αD helix of TLR4 blocked adapter recruitment to TLR4. A peptide (2R9) derived from the αD helix of TLR2, which shares about 40% sequence identity and structural similarity between TLR4 and TLR2, inhibited the recruitment of MAL to TLR2. Therefore, the present inventors predicted that the peptides composed of the entire αC helix (including aa P 155-T 166) could maintain the helical structure and would be sufficient to inhibit MAL-TIR assembly as well as other MyD88-dependent TLR signaling independent of the entire TIR domain.

The present inventors designed a decoy peptide (hereinafter defined as "MAL/MyD88 inhibitory peptide 2 (MIP2)") having a broad TLR inhibition potential using the MAL-αC interface knowledge drug pharmacophore model. MIP2 blocked MyD88 and TRIF-dependent LPS-induced TLR4 signaling in murine and human cell lines. MIP2 showed a therapeutic potential in psoriasis, SLE, and RA disease models. Characteristic serological and histological biomarkers were remarkably restored, and disease symptoms were substantially improved. Taken together, these *in vitro* and *in vivo* studies suggest that MIP2 has broad specificity of TLR and is effective in regulating autoimmune diseases and/or inflammatory diseases induced by microorganisms, environment, or genetic factors.

In addition, the present inventors investigated the interference of ligand-induced TLR signaling by conjugating a peptide containing the αC helix of MAL with the reported cell penetrating peptide. According to the initial SEAP analysis, while CPP was an efficient carrier of a peptide, a PFV-conjugated peptide did not show a significant effect (FIG. 1C). The present inventors assumed that it was due to the low cell permeability of the PFV-conjugated peptide or that CPP had an additional TLR-inhibitory effect. According to previous studies independent of TLR and further investigation by the present inventors, there was no TLR-inhibiting effect when CPP was used alone.

Lactam bridges and disulfide bonds have been widely used to limit structural freedom and improve receptor selectivity and binding affinity of therapeutic peptides (Harrison, R. S. et al., Proc Natl Acad Sci USA 107, 11686-11691 (2010)). Therefore, the present inventors applied a disulfide structural limitation to MIP2 (or MIP3). MIP3 showed an unexpected cytotoxic effect, but MIP2 did not show cytotoxicity at the same concentration (FIG. 2A). In order to confirm that C-terminal residues, particularly L165, are important for the TLR-inhibitory effect of MIP2, derivatives were prepared by substitution of residues. MIP2-1 (L165H and other substitutions) caused the loss of the potential to inhibit LPS-stimulated TLR4 signaling. However, the effect was maintained in MIP2-2, and thus the loss of the effect was not due to C157A (FIGS. 2D and 2E).

The TLR-inhibitory effect of MIP2 was further evaluated in human and murine macrophages. MIP2 not only exhibited a broad TLR-inhibitory effect, but also inhibited MyD88 and TRIF-dependent pathways of TLR4 in LPS-stimulated RAW264.7 cells and THP1-derived macrophages (FIGS. 3 and 4). This may be partly explained by the fact that MIP2 consists of the αC helix of MAL, which has the potential to bind MAL, TLR4-TIR, and MyD88-TIR (FIG. 1A). In addition, MAL and TRAM compete for binding to the overlapping interface of TLR4-TIR, and MIP2 is involved in both cases. A peptide partially overlapping with the C-terminus of MIP2 can interact with MyD88, excluding physical interactions between MyD88 and TRAM with TLR4. It was found that MIP2 possesses a broad range of TIR-domain binding ability and thus possesses a broad TLR-inhibition potential. NLRP3 induction and mature IL-1β release are associated with TLR-stimulation. Both MyD88- and TRIF-dependent TLR pathways can sensitize and activate the NLRP3 inflammasome. The present inventors confirmed that MIP2 suppressed the expression of NLRP3 and pro-IL-1β in ATP-activated THP-1 cells sensitized by LPS (FIG. 4C).

The relationship between the TLR signaling pathway and various human diseases, including SLE, RA, psoriasis, and sepsis, has been demonstrated by numerous data (Duffy, L. & O'Reilly, S. C., ImmunoTargets and therapy 5,69 (2016)). The therapeutic potential of MIP2 was evaluated in animal models of psoriasis, SLE and RA. MIP2 substantially alleviated the symptoms of IMQ-induced psoriasis in C57BL/6 mice and limited the relevant biomarkers (CD68, CD4, and IL17). MIP2 reduced the characteristics of SLE-associated serum markers (*e.g*., IL6, anti-dsDNA, ANA and urine albumin) and alleviated SLE-associated lymphadenopathy and splenomegaly. In addition, the treatment for 5-6 weeks completely cured the symptoms of arthritis in rats and restored the relevant parameters.

Accordingly, in one aspect, the present invention relates to a peptide represented by the amino acid sequence of SEQ ID NO: 1 or SEQ ID NO: 3.

In the present invention, the peptide may be characterized in that it inhibits the signaling pathway of any one or more TLRs (Toll-like receptors) selected from the group consisting of TLR4, TLR1/2, TLR2/6, TLR3, TLR7/8, TLR7, and TLR9. Preferably, the peptide may be characterized by inhibiting the TLR4 or TLR7 signaling pathway.

Ligand-induced dimerization of the TLR ectodomain induces rearrangement of the transmembrane and juxtamembrane regions that promote homotypic and heterotypic association of the TIR domain. As a result, it leads to recruitment of TIR-containing adapter molecules through cooperative interface sharing (Monie, T. P., et al., Immunol Rev 227, 161-175 (2009)). The complexity and ambiguous stoichiometry of the TIR-TIR interaction become the reasons for the lack of a generally accepted interface. It is generally accepted that the TIR-TIR interaction is an essential early-onset event for TLR signaling. For having an important role in TLR-signaling, degradation of the TIR-assembly has the potential to abolish TLR signaling in related immune complications. In summary, it can be seen through the results of *in vitro* and *in vivo* studies according to the present invention, the peptide of the present invention has a wide range of specificity for TLR inhibition, and that it would be effective in controlling autoimmune complications induced by microorganisms or environmental factors.

In the present invention, the peptide may be characterized in that it is derived from the αC helix of MAL (TIR domain adapter protein; TIRAP).

In the present invention, the peptide may be characterized in that it binds to a Toll/interleukin-1 receptor (TIR) domain or a TIR-containing adapter molecule.

The TIR domain may be a domain of TLR, in particular TLR4 signaling, in which the TIR-containing adapter molecule may be MAL (TIR domain adaptor protein; TIRAP), myeloid differentiation primary response 88 (MyD88), TRIF (TIR domain-containing adapter-inducing interferon β; TICAM-1), or TRAM (TICAM-2), but is not limited thereto.

As used herein, the term "peptide" refers to a linear molecule formed by combining amino acid residues with each other by a peptide bond. The peptide may be prepared according to a chemical synthesis method known in the art, and preferably according to a solid-phase synthesis technique, but the method is not limited thereto.

As used herein, the term "TLR4" refers to a protein that belongs to TLRs, which is a family of transmembrane proteins that function as a monitor for pathogen infection, and is a protein encoded by the TLR4 gene, and is also called cluster of differentiation 284 (CD 284). The TLR4 is very important for the activation of the innate immune system because it recognizes various pathogen-associated molecular patterns (PAMPs) including LPS of gram-negative bacteria.

As used herein, the term "TLR4 signaling pathway" refers to a signaling pathway through TLR4. TLR4 delivers signals by several adapter proteins, and the signaling pathway operates with TIRAP (also referred to as MAL) and MyD88, and TRAM and TRIF. TLR4-mediated signaling pathway induces the activation of MyD88-dependent and MyD88-independent signaling through various sub-signaling molecules. While the initiation of the MyD88-dependent pathway induces early-stage activation of NF-κB and secretion of pro-inflammatory cytokines (e.g., TNF-α and IL-6), the initiation of the MyD88-independent pathway induces activation of IRF3 and IRF7, secretion of IFNs, and late-phase activation of NF-κB (Park, B.S et al., Exp. Mol. Med. 45:e66, 2013; Nijland, R et al., Mar. Drugs 12:4260-4273, 2014). In addition, TLR4 induces activation of MAPKs, including ERK, JNK, and p38, and secretes inflammatory cytokines and IFNs (Achek, A et al., Arch. Pharmacal Res. 39: 1032-1049,2016; Kawai,T et al., Nat. Immunol. 11:373-384, 2010). In macrophages, stimulation of TLR4 by associated ligand induces COX2 and iNOS, induces NO production, and produces mitochondrial and intracellular ROS (Kim, J.Y et al., Eur. J. Pharmacol. 584:175-184, 2008; Liu, K.-L. et al. J. Agric. Food Chem. 54:3472-3478, 2006; West, A.P. et al. Nature 472:476, 2011).

In the present invention, the TLR4 signaling pathway may be characterized in that it is induced by lipopolysaccharide (LPS).

As used herein, the term "inhibition" refers to a phenomenon in which biological action or activity is reduced due to deficiency, disharmony, and many other causes, and this phenomenon may be a phenomenon of partially or completely blocking, decreasing, or preventing activity of TLRs, delaying activation thereof, or inactivating or downregulating TLRs.

In the present invention, blockage of the TLR4 signaling pathway by the peptide may result in inhibition of secretion of IL-6 and TNF-α; inhibition of activity of NF-κB; inhibition of expression of NLRP3 and pro-IL-1β; inhibition of phosphorylation of IRF3 and IRF7 and inhibition of expression of ATF3; inhibition of expression of iNOS and COX2; and inhibition of release of NO and production of intracellular ROS, and the peptide may be characterized in that it inhibits both MyD88- and TRIF-dependent TLR4 signaling pathways.

In another aspect, the present invention relates to a fusion peptide in which a cell penetrating peptide is bound to the peptide.

In the present invention, the fusion peptide may be characterized in that it inhibits TLR4 or TLR7 signaling pathway, and the TLR4 signaling pathway may be characterized in that it is induced by LPS.

In addition, in the present invention, blockage of the TLR4 signaling pathway by the fusion peptide may result in inhibition of secretion of IL-6 and TNF-α; inhibition of activity of NF-κB; inhibition of expression of NLRP3 and pro-IL-1β; inhibition of phosphorylation of IRF3 and IRF7 and inhibition of expression of ATF3; inhibition of expression of iNOS and COX2; and inhibition of release of NO and production of intracellular ROS, and the fusion peptide may be characterized in that it inhibits both MyD88- and TRIF-dependent TLR4 signaling pathways.

As used herein, the term "cell penetrating peptide (CPP)" refers to a kind of signal peptide, which is a peptide that is a combination of specific amino acid sequences used for the purpose of delivering high molecular materials (*e.g*., proteins, DNA, RNA, *etc.)* into cells. Until now, the cell penetrating peptide has been used for intracellular delivery of various low-molecular compounds, and other high molecular materials (*e.g*., proteins, peptides, RNA, DNA, *etc.)*

The fusion peptide of the present invention uses a cell penetrating peptide, and the cell penetrating peptide is not particularly limited as long as it has a characteristic of entering into a cell by endocytosis mechanism, but preferably, the cell-penetrating peptide may be selected from the cell-penetrating peptides listed in Table 1 or variants thereof, and used. More preferably, the cell-penetrating peptide may be characterized in that they may be represented by an amino acid sequence of SEQ ID NO: 6.

**[Table 1]**

| Cell penetrating peptides | | |
|---|---|---|
| **Peptide** | **Origin** | **Sequence (5'->3')** |
| Penetratin | Drosophila Antennapedia homeodomain | RQIKIWFQNRRMKWKK (SEQ ID NO: 6) |
| TAT(₄₈₋₆₀) | Human immunodeficiency virus type 1 (HIV-1) TAT | GRKKRRQRRRPPQ |
| pVEC | VE-Cadherin(615-632) | LLIILRRRIRKQAHAHSK |
| Transportan 10/TP10 | Galanin-Lys-mastoparan | GWTLNSAGYLLGKINLKALAA LAKKIL |
| MPG | A hydrophobic domain from the fusion sequence of HIV gp41 and NLS of SV40 T-antigen | GALFLGFLGAAGSTMGAWSQP KKKRKV |
| Pep-1 | NLS from Simian Virus 40 large T antigen and reverse transcriptase of HIV-1 | KETWWETWWTEWSQPKKKRK V |
| MAP | Amphipathic model peptide | KLALKLALKALKAALKLA |
| R₆/W₃ | Based on penetratin | RRWWRRWRR |
| Polyarginine (R₉,R₈) | Positively charged sequence | Rₙ, n = 8 or 9 |
| VP22 | Herpes simplex virus | NAKTRRHERRRKLAIER |
| YTA2 | MMP cleavage site as seeding sequence | YTAIAWVKAFIRKLRK |
| YTA4 | MMP cleavage site as seeding sequence | IAWVKAFIRKLRKGPLG |
| M918 | The tumor suppressor protein p14ARF | MVTVLFRRLRIRRACGPPRVRV |
| CADY | Derived from PPTG1 peptide, W and charged amino acids | GLWRALWRLLRSLWRLLWRA |
| SAP | Designed based on a natural protein of maize, γ-zein VHL (PPP)₈ | (VRLLPPP)₃ |
| SAP(E) | Design inspired by SAP; Arg residue replaced by Glu | Ac-CGGW(VELPPP)₃ |
| CyLoP-1 | Derived from crotamine toxin found in snake venom, crot(27-39) | CRWRWKCCKK |
| gH 625 | Based on the 625-644 residues of the glycoprotein HSV 1 | HGLASTLTRWAHYNALIRAF |
| GALA | Glu-rich an containing His (imidazole group) in order to be pH responsive (endosomes) (Designed to efficiently escape endosomes) | WEAALAEALAEALAEHLAEAL AEALEALAA |
| CADY | Designed; based on chimeric peptide carrier PPTG1 derived | Ac- GLWRALWRLLRSLWRLLWRA- |
| | from the fusion peptide JTS 1 | cysteamide |
| L17E | Inspired by the spider venom M-lycotoxin | IWLTALKFLGKHAAKHEAKQQ LSKL |
| MPPs | Designed to contain un-natural, cyclohexylalanine (Fₓ) residues and to have differential intracellular localization | Mitochondria-penetrating peptides (example: FxrFxKFxrFxK) |
| RR₅-APP RR₄-APP RR₃-APP | Small proteins (36-residue polypeptides) | RPRRPRRPRRPGRRAPVEDLIRF YNDLQQYLNVVTRHRYC RRPRRPRRPGRRAPVEDLIRFY NDLQQYLNVVTRHRYC GPRRPRRPGRRAPVEDLIRFYN DLQQYLNVVTRHRYC |
| TATp-D | Analogue of TAT | Ac-GRKKRRQRRRPPQ-K-K ; Ac-GRKKRRQRRRPPQ |
| Cyclic Tat | Lys- and Glu- amino acids added to the linear Tat sequence to obtain a ring with the same overall charge as the native form | c [K-rRrQrRkKrG-E] c |
| K₁₀(QW)₆ | The design was based on combining W and K at the primary structure level to obtain self-assembly into a variety of nano structure s | KKKKKKKKKQWQWQWQWQ WQW |
| TAT (₄₉₋₅₇) | HIV-1 TAT protein | RKKRRQRRR |
| DPV1047 | Chemically synthesized | VKRGLKLRHVRPRVTRMDV |
| ARF(₁₋₂₂) | p14ARF protein | MVRRFLVTLRIRRACGPPRVRV |
| BPrPr₍₁₋₂₈) | N terminus of unprocessed bovine prion protein | MVKSKIGSWILVLFVAMWSDV GLCKKRP |
| p28 | Azurin | LSTAADMQGVVTDGMASGLD |
| | | KDYLKPDD |
| VT5 | Chemically synthesized | DPKGDPKGVTVTVTVTVTGKG DPKPD |
| C105Y | α1-Antitrypsin | CSIPPEVKFNKPFVYLI |
| PFVYLI | Derived from synthetic C105Y | PFVYLI (SEQ ID NO: 7) |
| Pep-7 | CHL8 peptide phage clone | SDLWEMMMVSLACQY |

Meanwhile, among the cell-penetrating peptides in Table 1, Transportan includes those used in the form of the following variants:
AGYLLGKINLKALAALAKKIL-NH₂ (TP10, PepFect 3),
AGYLLGKINLKALAALAKKIL-NH₂ (TP10, PepFect 6),
AGYLLGKLLOOLAAAALOOLL-NH₂(TP10, PepFect 14),
AGYLLGKTNLKALAALAKKIL-NH₂ (NickFect 1),
AGYLLGKTNLKALAALAKKIL-NH₂ (NickFect 2), and
AGYLLGKTNLKALAALAKKIL-NH₂ (Nickfect 3).

In an embodiment of the present invention, the experiment was performed by selecting the Penetratin sequence (RQIKIWFQNRRMKWKK; SEQ ID NO: 6) and PFVYLI (SEQ ID NO: 7) among the cell-penetrating peptides in Table 1. It will be apparent to those skilled in the art that effects similar to those of the present invention would appear even when other cell penetrating peptides are fused with the peptide of the present invention in addition to the cell penetrating peptides actually used. Preferably, this experiment may be characterized by fusing a cell penetrating peptide comprising an amino acid sequence represented by SEQ ID NO: 6.

In the present invention, the fusion peptide may be characterized in that a cell penetrating peptide is bound to the N-terminus or C-terminus of the peptide. Preferably, the fusion peptide may be characterized in that a cell penetrating peptide is bound to the N-terminus of the peptide, but is not limited thereto.

In the present invention, the fusion peptide, in which a cell penetrating peptide is bound to the N-terminus of the peptide, may preferably be characterized in that it consists of the amino acid sequence of SEQ ID NO: 8. In addition, the variant with the amino acid sequence may also be included within the scope of the present invention. Specifically, the variant may include all of the peptides having a sequence homology, to SEQ ID NO: 8, respectively, of at least 70%, preferably at least 80%, more preferably at least 90%, even more preferably at least 95%, still more preferably at least 98%, and most preferably at least 99%. The term "homology" is intended to refer to a degree of similarity with a wild type amino acid sequence or a wild type nucleic acid sequence.

In another aspect, the present invention relates to a TLR antagonist comprising the peptide or the fusion peptide.

In the present invention, the TLR may be any one or more selected from the group consisting of TLR4, TLR1/2, TLR2/6, TLR3, TLR7/8, TLR7, and TLR9.

As used herein, the term "antagonist" refers to a molecule which partially or completely inhibits the effect of another molecule (*e.g*., a receptor or intracellular mediator) by any mechanism.

As used herein, the term "TLR antagonist" refers to a material which can directly/indirectly or substantially interfere, reduce, or inhibit the biological activity of TLR, preferably, the peptide, which is reactive with TLR, binds directly to the TLR or TIR domain, neutralizes the activity of the TLR, and block the TLR signaling pathway, can induce a decrease in the activation of NF-κB and MAPKs, thereby reducing the secretion of inflammatory cytokines, NO, and ROS.

According to an embodiment of the present invention, the peptide represented by SEQ ID NO: 1 of the present invention has an excellent effect of inhibiting the secretion of IL-6, NO, and ROS and the activation of NF-κB and MAPKs by inhibiting the TLR4 signaling pathway induced by LPS, and thus can be usefully used as a composition for preventing or treating autoimmune diseases and inflammatory diseases caused by the TLR4 signaling pathway.

Considerable efforts have been made to develop TLR-inhibiting molecules that can target ligand-binding domains. Despite many efforts to target protein-protein interfaces (PPIs) with small molecules, selection of effective candidates for biologically important PPIs still remain as a challenge. The insolubility of small molecules to the TIR-domain may be due in part to the lack of sufficient structural information, the flat protein-protein interface, and the poorly defined pockets. Although peptide-drugs are susceptible to enzymatic cleavage and have low bioavailability (Newland, A. et al., British journal of haematology 135, 547-553 (2006)), provide low toxicity and improved target selectivity compared to small molecules. The development of peptides or peptide-analogs is a key method for expanding the "druggable genome" by specifically targeting PPIs that cannot be used as pharmaceutical drugs (Hopkins, A. L. & Groom, C. R., Nat Rev Drug Discov 1, 727-730 (2002)).

In another aspect, the present invention relates to a composition for preventing or treating autoimmune diseases comprising the peptide or the fusion peptide.

As used herein, the term "autoimmune disease" refers to a disease that is caused by a process in which a phenomenon is generated where a problem occurs in inducing or maintaining self-tolerance, which causes immune responses to self-antigens, such that the body's own tissue is attacked thereby. The self-tolerance refers to immunologic unresponsiveness that does not harmfully respond to antigenic materials constituting the self. The autoimmune disease of the present invention is characterized in that it is selected from the group consisting of psoriasis, rheumatoid arthritis, psoriatic arthritis, experimental autoimmune arthritis, asthma, Crohn's disease, multiple sclerosis,experimental autoimmune encephalomyelitis, myasthenia gravis, thyroiditis, experimental form of uveitis, Hashimoto thyroiditis, primary myxedema, thyroid poisoning, malignant anemia, autoimmune atrophic gastritis, Addison's disease, premature menopause, male infertility, childhood diabetes, Goodpasture syndrome, common pemphigus, pemphigoid, sympathetic ophthalmitis, lens uveitis, autoimmune hemolytic anemia, idiopathic leukocytopenia, primary biliary cirrhosis, chronic active hepatitis, latent cirrhosis, ulcerative colitis, Sjogren's syndrome, scleroderma, Wegener's granulomatosis, polymyositis, skin myositis, discoid lupus, and systemic lupus erythematosus, but is not limited thereto.

The composition for preventing or treating autoimmune diseases according to the present invention may include a pharmaceutically effective amount of the peptide or fusion peptide alone, or may include one or more pharmaceutically acceptable carriers, excipients, or diluents. The pharmaceutically effective amount refers to an amount sufficient to prevent, improve, and treat symptoms of autoimmune diseases.

The term "pharmaceutically acceptable" refers to one which is physiologically acceptable and normally does not cause gastrointestinal disorders, allergic reactions (*e.g*., dizziness), or similar reactions when administered to humans. Examples of the carrier, the excipient, and the diluent may include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinyl pyrrolidone, water, methylhydroxy benzoate, propylhydroxy benzoate, talc, magnesium stearate, and mineral oil. In addition, the composition of the present invention may further include a filler, an anticoagulant, a lubricant, a wetting agent, a fragrance, an emulsifier, a preservative, *etc.*

In addition, the pharmaceutical composition of the present invention may include at least one kind of known active ingredient having a therapeutic effect on autoimmune diseases, along with the peptide or fusion peptide.

The pharmaceutical composition of the present invention may be formulated using methods known in the art so that the pharmaceutical composition can provide rapid, sustained, or delayed release of the active ingredients after being administered to mammals excluding humans. The formulation may be in the form of powders, granules, tablets, emulsions, syrups, aerosols, soft or hard gelatin capsules, sterile injectable solutions, and sterile powders.

The pharmaceutical composition of the present invention may be administered through various routes including oral, transdermal, subcutaneous, intravenous, and intramuscular routes, and the dose of the active ingredients may appropriately be selected according to various factors (*e.g*., the route of administration, the patient's age, sex, body weight, severity of the patient's condition, *etc.).* The composition for preventing or treating autoimmune diseases according to the present invention may be administered in combination with known compounds having an effect of preventing, improving, or treating symptoms of autoimmune diseases.

In still another aspect, the present invention relates to a composition for preventing or treating inflammatory diseases comprising the peptide or the fusion peptide.

As used herein, the term "inflammatory disease" refers to a disease which is caused by inflammatory materials (inflammatory cytokines) (e.g., TNF-α, IL-1, IL-6, prostaglandin, leukotriene, and NO), which are secreted by immune cells (*e.g*., macrophages) in a case where the immune system is excessively accentuated due to harmful stimuli (*e.g*., inflammation-inducing factors, irradiation, *etc.).* The inflammatory disease of the present invention is characterized in that it is selected from the group consisting of insulin-dependent diabetes, eczema, allergy, atopic dermatitis, acne, atopic rhinitis, pneumonia, allergic dermatitis, chronic sinusitis, contact dermatitis, seborrheic dermatitis, gastritis, gout, gouty arthritis, ulcer, chronic bronchitis, ulcerative colitis, ankylosing spondylitis, sepsis, vasculitis, bursitis, lupus, rheumatoid polymyalgia, temporal arteritis, solid tumor, Alzheimer's disease, atherosclerosis, obesity, viral infection, and nonalcoholic steatohepatitis, but is not limited thereto.

Since the composition for preventing or treating inflammatory diseases includes a pharmaceutical preparation containing the above-described peptide as an active ingredient, the description of overlapping contents with regard to the composition of the present invention will be omitted herein.

In still another aspect, the present invention relates to a method for preventing or treating autoimmune diseases comprising administering the peptide or the fusion peptide.

In still another aspect, the present invention relates to the use of the peptide or the fusion peptide for preventing or treating autoimmune diseases.

In still another aspect, the present invention relates to the use of the peptide or the fusion peptide in preparation of a medicament for preventing or treating autoimmune diseases.

In still another aspect, the present invention relates to a method for preventing or treating inflammatory diseases comprising administering the peptide or the fusion peptide.

In still another aspect, the present invention relates to the use of the peptide or the fusion peptide for preventing or treating inflammatory diseases.

In still another aspect, the present invention relates to the use of the peptide or the fusion peptide in preparation of a medicament for preventing or treating inflammatory diseases.

Since the method for prevention or treatment, use thereof, and utilization employs "the peptide or the fusion peptide or a composition comprising the same", the description of overlapping contents will be omitted herein.

### Mode for the Invention

Hereinafter, the present invention is explained in detail by Examples. These Examples are only for illustrating the present invention, and it will be apparent to those of ordinary skill in the art that the scope of the present invention is not to be construed as being limited by these Examples.

### Example 1: Materials and Methods

### Example 1-1: Peptide synthesis, reagents, and optimization of cell lines

As determined by reversed-phase high performance liquid chromatography (HPLC; Shimadzu Prominence), peptides were synthesized by Biostem (Ansan, Korea) with a purity of 97.09% (MIP1), 97.17% (MIP2), and 95.1% (MIP3). For all of the peptides, Shiseido Capcell pak C18 column (4.6 mm × 50 mm) was used, and the peptides were detected at 220 nm at a flow rate of 1 mL/min with a gradient of 10-60% acetonitrile in 0.1% trifluoroacetic acid (TFA)-water. The molecular weights of MIP1, MIP2, and MIP3 were measured by Shimadzu LCMS-2020 and were 2,182.6 Da, 3,710.5 Da, and 3,680.5 Da, respectively. LPS (*Escherichia coli* 0111: B4) and adenosine triphosphate (ATP) were purchased from Sigma-Aldrich. PAM₃CSK₄ (TLR1/2), Poly(I:C) (TLR3), Imiquimod (IMQ; R837, TLR7), R848 (TLR7/8), and CpG-ODN (TLR9) were purchased from Thermo Fisher Scientific, Inc., and FSL-1 (TLR2/6) was purchased from InvivoGen (San Diego, CA, USA).

HEK-Blue^{™} hTLR4 cells (InvivoGen, San Diego, CA, USA) and RAW264.7 cells were cultured in high sugar Dulbecco's modified Eagle's medium (DMEM), which does or does not contain 1% penicillin/streptomycin solution and 10% fetal bovine serum (FBS) (Thermo Fisher Scientific, Inc., Waltham, MA, USA) and 0.2% normocin (InvivoGen, San Diego, CA, USA). THP-1 cells were cultured in RPMI 1640 medium supplemented with 1% penicillin/streptomycin solution and 10% FBS, and were induced to differentiate into macrophages for 24 hours using 80 nM phorbol 12-myristate 13-acetate (PMA; Sigma-Aldrich Co., St. Louis, MO, USA). All cells were cultured under humid conditions at 5% CO₂, 37°C (Thermo Fisher Scientific, Inc.), and the medium was replaced after 18 hours of incubation.

### Example 1-2: Cell viability assay

HEK-Blue^{™} hTLR4 cells were dispensed at a density of 5 × 10⁴ cells/well, and RAW264.7 cells and THP-1 cells were dispensed thereinto at a density of 2 × 10⁵ cells/well. All cells were cultured overnight in 96-well plates (BD Biosciences, San Jose, CA, USA). Cell viability was determined using colorimetric 1-(4,5-dimethylthiazol-2-yl)-3,5-diphenylformazan (MTT) assay (Sigma-Aldrich) and/or MTS (3-(4,5-dimethylthiazol-2-yl)-5-(3-carboxymethoxyphenyl)-2-(4-sulfophenyl)-2H-tetrazolium) (Promega Madison, WI, USA), which was performed as described previously (Kwon, H. K. et al., Toxicological sciences: an official journal of the Society of Toxicology 148, 204-219 (2015)).

### Example 1-3: Assay for SEAP activity

HEK-Blue^{™} hTLR4 cells were dispensed into 24-well plates (BD Biosciences) at a density of 2 × 10⁵ cells/well and cultured overnight. The cells were treated with different concentrations of MIPs in the presence and/or absence of LPS (100 ng/mL). A portion of the supernatant (200 µL) from the treated cells was transferred to a microcentrifuge tube and heated at 65°C for 10 minutes using a heating block (FINEPCR Co., Seoul, Korea). Then, the supernatant was transferred to a new 96-well plate (BD Biosciences), and SEAP production was detected using HEK-Blue^{™} detection kit (InvivoGen, San Diego, CA, USA). Absorbance was measured at 620 nm using a microplate reader spectrophotometer system (Molecular Devices Inc., Silicon Valley, CA, USA).

### Example 1-4: ELISA

THP-1-derived macrophages and RAW264.7 cells were dispensed into 96-well plates (BD Biosciences) at a density of 2 × 10⁵ cells/well and cultured overnight. After 24 hours of treatment, IL-6 secretion was analyzed using Mouse IL-6 ELISA MAX^{™} Deluxe (BioLegend, San Diego, CA, USA), and TNF-α production was evaluated using the Mouse TNF alpha ELISA Ready-SET-Go!^{®} kit (eBioscience, San Diego, CA, USA). Then, the plate was analyzed at each wavelength using a microplate spectrophotometer system (Molecular Devices).

### Example 1-5: Protein quantification and Western blot analysis

Total-protein extraction was performed using M-PER Mammalian Protein Extraction Reagent (Thermo Fisher Scientific, Inc.). The protein concentration was measured with a bicinchoninic acid (BCA) assay kit (Sigma-Aldrich). Western blot analysis including gel electrophoresis and development was performed using Mini-PROTEAN Tetra Cell and mini trans-blot electrophoretic transfer cell system (Bio-Rad Laboratories, Hercules, CA, USA) (Kwon, H. K., et al., Sci Rep 5, 15623 (2015)). The membrane was immunoblotted with primary antibodies by gentle shaking overnight at a temperature of 4°C (1:500-1000), in which the primary antibodies were antibodies against p-p65, p-JNK, JNK, p-IRF3, p-IRF7, p-ERK, ERK, p-p38, p38, Iκ-Bα, IL1β, NLPR3, and p-IRAK4 (Cell Signaling Technology Inc., Danvers, MA, USA); p-ERK, p-p38, JNK, ATF3, COX2, and β-actin (Santa Cruz Biotechnology Inc., Dallas, TX, USA); and iNOS (BD Biosciences). Then, the membrane was thoroughly washed with phosphate buffered saline with Tween 20 (PBST) and incubated with peroxidase-conjugated anti-mouse or anti-rabbit IgG antibody (1:1000) for 2 hours. Proteins were detected with the SuperSignal West Pico ECL solution (Thermo Fisher Scientific, Inc.) and the detected proteins were visualized with ChemiDoc^{™} Touch Imaging System (Bio-Rad Laboratories).

### Example 1-6: Evaluation of intracellular NO and ROS and analysis of NO secretion

1 × 10⁶ RAW264.7 cells were dispensed into a 6 cm culture dish (SPL Life Sciences, Pochun, Korea) and cultured overnight. After LPS stimulation and MIP2 treatment, intracellular nitric oxide (NO) and reactive oxygen species (ROS) were quantified using DAF-FM and DCF-DA dyes (Thermo Fisher Scientific, Inc.) (Kwon, H. K., et al., Sci Rep 5, 15623 (2015)). The fluorescence intensity of the cells was analyzed using the FACSAria III instrument with Diva software (BD Biosciences). To evaluate extracellular NO production, RAW264.7 cells were dispensed into 96-well plates (BD Biosciences) at a density of 2 × 10⁵ cells/well, and cultured overnight. NO secretion was measured with the Nitric Oxide Detection Kit (iNtRON Biotechnology Inc., Seongnam, Korea) (Kwon, H. K. et al., Toxicological sciences: an official journal of the Society of Toxicology 148, 204-219 (2015)). Absorbance was measured at 550 nm using a microplate spectrophotometer system (Molecular Devices).

### Example 1-7: Confocal microscopy assay

RAW 264.7 cells were dispensed into 24-wellplates (BD Biosciences) at a density of 2 × 10⁵ cells/well and the cells were treated with MIP2 (50 µM) for one hour before LPS stimulation (100 ng/mL). The cells were fixed with a 3.7% formaldehyde solution (Sigma-Aldrich) and immersed in a 0.2% Triton X-100 solution (AMRESCO, Solon, OH, USA) for 15 minutes. Then, the cells were washed with PBS and blocked using a 2% BSA solution (Thermo Fisher Scientific, Inc.). Then, these cells were cultured with anti-p-p65 antibodies (1:1000; Santa Cruz Biotechnology Inc.) for two hours and washed thoroughly with PBS. Then, the cells were cultured with Alexa Fluor 546 secondary antibodies (Invitrogen, Carlsbad, CA, USA) for one hour and washed three times with PBS. Nuclei were stained using the Hoechst 33258 solution (5 µM; Sigma-Aldrich). Fluorescence intensity was measured with a confocal microscope (LSM-700; Carl Zeiss Microscopy GmbH, Munich, Germany) and images were analyzed using Zen 2009 software.

### Example 2: Animal Experiment Procedure

### Example 2-1: Mouse model with psoriasis

6-7 week old C57BL/6 mice were purchased from Orient Bio Inc. (Seongnam, Korea). The mice were housed under specific pathogen-free conditions and provided *ad libitum* with a standard laboratory diet (STD). All the animal experiments were approved by the Institutional Animal Care and Use Committee (IACUC) of Ajou University (Approval No. 2017-0002). 62.5 mg of Aldara cream (including 5% IMQ; Aldara, 3M Pharmaceutical LLC.) was applied for five consecutive days, resulting in symptoms similar to psoriasis. The normal group was not treated with IMQ but only injected with PBS. The mice were administered with MIP2 (1 nmol/g, 10 nmol/g, or 20 nmol/g) or PBS (control) one day before applying the Aldara cream (5%). The mouse used as a positive control was administered with MTX (10 µg/g; Cat. No. M9929, Sigma-Aldrich) one day before the application of the IMQ cream. After 5 days, the mice were euthanized by respiratory anesthesia, and samples of skin lesions and spleen were collected for histological examination. To evaluate the degree of inflammation of the skin on the back, an objective scoring system was developed based on the clinical PASI. Factors such as erythema, scaling, wrinkles, and thickening were independently scored from 0 to 4: 0, none; 1, a little; 2, middle; 3, significant; 4, very prominent. Erythema, scaling, and thickness were scored under the close supervision of an experienced researcher. The cumulative score (erythema+scaling+thickening, grade 0 to grade 12) represents the severity of psoriasis symptoms.

A skin sample obtained from the dorsal lesion of each mouse was fixed in 4% paraformaldehyde solution, embedded in paraffin, and sectioned at a thickness of 7 µm on a glass slide. Sections were stained with H&E to determine the thickness of the epidermis and dermis. The thickness of the skin was measured using a Leica DMi8 fluorescence microscope which utilizes a Leica LAS X Hardware Configurator. IMQ-mediated inflammation in the skin was evaulated using a mouse-specific HRC/DAB Detection IHC Kit (Abcam, Cat. No. AB64259) with primary antibodies recognizing CD68, CD4, and IL-17.

### Example 2-2: Mouse model with SLE

Six female wild-type (C57BL/6) and lupus-prone mice (MRL/lpr) with initial weights of 18-20 g and 38-40 g, respectively, were purchased from Jackson Laboratory (Bar Harbor, ME, USA). All animal experiments were reviewed and approved by the IACUC, Ajou University Medical Center (Approval No. 2017-0022). The mice were acclimatized for one week, and then bred in pathogen-free conditions according to the approved guidelines of Ajou University Medical School. While the mice in the vehicle group were intraperitoneally (ip) injected with PBS, others were injected with 10 nmol/g (in PBS) of MIP2 daily for 20 days (5 times a week for 4 weeks), and their weights were monitored daily. The mice were euthanized at the end of the experiment, and blood, urine, and tissues were collected. After incubating for one hour, blood samples were collected in serum separation tubes, centrifuged at 3,000 rpm for 10 minutes at 20°C, and stored at -80°C. The collected urine samples were immediately stored at -80°C, and the tissue samples were thoroughly washed with PBS and immersed in an RNA stabilization solution (Qiagen Sciences, Maryland, MD, USA). The concentrations of anti-nuclear antibodies, anti-dsDNA antibodies, IL-6, and C3 complements were analyzed with an ELISA kit (MyBiosource, San Diego, CA). In addition, urine albumin levels of the mice were analyzed using the Mouse Albumin ELISA Kit (41-ALBMS-E01, Alpco Diagnostics, Salem, NH, USA). Kidney tissue was collected, fixed in 4% paraformaldehyde (PFA), and refrigerated at 4°C overnight. Then, the fixed tissue was embedded in paraffin, sectioned to a thickness of 2-4 µm, and stained with Periodic Acid-Schiff (PAS) or H&E reagent. Three independent measurements were performed per sample.

### Example 2-3: Model with collagen-induced arthritis (CIA)

Four-week-old male Lewis rats were injected with 250 µL (including 0.5 mg of collagen) of a mixed solution (bovine type II collagen and complete Freund's adjuvant in a 1:1 ratio) to induce RA. After 3 weeks of disease development, edema and erythema were observed individually in the ankle bones, ankle joints, knee joints, and paws of the rats. The experiment was performed according to the guidelines approved by the Animal Experimentation Committee of Ajou University School of Medicine (Approval No. 2013-0070). The rats were divided into three groups (each group included three rats): normal (non-CIA, injected with PBS only), untreated CIA, and MIP2-treated CIA. CIA rats in the treatment group were individually injected with 100 µL of MIP2 at a concentration of 20 pmol/g (5.94 µg/joint) into the knee joint.

To determine the ankle diameters and arthritic index (AI) scores, hind paws of individual rats in each group were observed on the 1^{st}, 2^{nd}, 3^{rd}, 4^{th}, 5^{th}, and 6^{th} week of the treatment regimen. The ankle diameter was measured using Vernier calipers, and the AI was scored after a blind test as follows: 0, no edema or erythema; +1, slight edema and erythema; +2, moderate edema and shackles; +3, a limited use of the joints to the metatarsal and edema with enlarged symptoms; +4, joint stiffness and excessive distension due to severe symptoms associated with the whole hind paws. The results of three independent measurements of ankle diameter and AI score were expressed as mean ± standard deviation (SD).

### Example 2-4: Mouse model with Nonalcoholic steatohepatitis (NASH)

Twenty four nine-week-old male wild-type (C57BL/6J) mice (initial weight: 21-28 g) were purchased from Japan SLC, Inc. (Shizuoka, Japan). From 10 weeks of age, the mice were divided into four groups of six mice. The control group with normal chow diet, the MCD group with MCD diet, the MIP2 prevention group with MCD diet and MIP2 were treated from the beginning, whereas the MIP2 treatment group with MCD diet and MIP2 were treated from the 3^{rd} week. The mice in the control group or the MCD group were injected intraperitoneally (i.p.) with PBS, whereas others were administered MIP2 at 10 nmol/g (in PBS) seven times a week for six weeks, and their body weights were monitored daily. At the end of the experiment, the mice were euthanized, and blood and liver tissues were collected. After one hour of incubation, blood samples were collected in serum separation tubes, centrifuged at 3,000 rpm, 20°C, for 10 minutes, and stored at -80°C. Liver lysates were separated with RIPA buffer (Biosesang, R2002, Korea) according to the manufacturer's instructions. The triglyceride levels of liver lysates were measured using a triglyceride quantification colorimetric kit (Biovision K622-100, Inc.). The cholesterol levels of liver lysate were measured using a Total Cholesterol and Cholesteryl Ester colorimetric kit (Biovision K603-100, Inc.). The qRT-PCR analysis was performed using an ABI Prism 7500 qRT-PCR system (Applied Biosystems, Foster City, CA, USA). The gene expression was detected with SYBR Premix Ex Taq (Takara RR420A, Inc.), normalized to the reference gene GAPDH by the delta-delta Ct method (2^{-ΔΔCt}), and relative gene expression levels were measured.

All experiments were performed at least three times, and each result was expressed as mean ± SD. One-way ANOVA with post-hoc analysis was used for all statistics (^{∗}P < 0.05; ^{∗∗}P < 0.001; ###P < 0.0001 compared to the MCD group). All animal experiments were reviewed and approved in Biotoxtech Co., Ltd. (Cheongju, South Korea) by IACUC, Ajou University Medical Center (Approval No. 180798).

### Example 3: Peptide design and in silico (computer programming in virtual experiments) analysis

Two interfaces for MAL-TIR dimers (*i.e.,* symmetric and asymmetric interfaces), have been proposed through crystallography. These findings are supported by a recent cryo-EM study which reports that asymmetric and symmetric interfaces are actually intra- and inter-chain interfaces of MAL-MAL protofilaments, respectively. An in-depth analysis of MAL symmetry or interchain interfaces stresses the pivotal role of αC in the MAL-MAL, MAL-TLR4, and MAL-MyD88 TIR-TIR interactions. Neither crystallization studies nor cryo-EM suggest the involvement of D154 at the MAL-TIR interface. Interface analysis revealed that D154 is present at the distal N-terminus of αC and does not directly interact with adjacent MALs (FIGS. 1A and 1B). In the NF-κB reporter analysis, Lin *et al.* reported that W156A, Y159A, and L165A mutations, but not D154A, have a significant effect on NF-κB signaling. In the same study, it was confirmed that D154A did not affect the MAL-TLR4-TIR and MAL-Myd88-TIR interactions. However, W156A and Y159A showed significant effects. In addition, Valkov *et al.* in their MAL structural study, found out through alanine scanning that residues 162-166 of MAL not only participate in MAL self-association, but also play an important role in MAL-MyD88 binding. Taken together, these findings suggest that the spanning of residues from P155 to T166 in the MAL-αC is important for the TIR-TIR interaction. Therefore, the αC binding interface in MAL, TLR, and MyD88 possesses several common pharmacophores that may be involved by αC itself or its analogs.

A dodecapeptide (a MAL/MyD88 inhibitory peptide (MIP)) was designed from the αC helix, and its structural stability and cell permeability were examined. To facilitate the penetration of MIP into a cell, the peptide was conjugated with PFVYLI, which is a reported cell penetrating peptide (hereinafter "PFV" (Rhee, M. & Davis, P., J Biol Chem 281, 1233-1240 (2006))) and classified as MIP1, or alternatively, it was classified as MIP2 by conjugation with penetratin (CPP (Derossi, D., et al., J Biol Chem 269, 10444-10450 (1994))).

In order to maintain the helicity and structural stability of MIP2, after a safe (FIG. 2A) and effective initial evaluation (FIG. 1B) *in vitro,* a structural limitation was applied by creating a disulfide bond between C157 and C161 (MIP3; the methionine at position 7 in the parent peptide is mutated to a cysteine) (FIG. 1B). However, MIP3 showed a cytotoxic effect at a given concentration (FIG. 2A).

In the TLR-inhibitory effect, the involvement of the C-terminal residue, particularly L 165, was confirmed by producing variants of MIP2 by residue substitution. MIP2-1 (L165H, C157A, and M161A mutations) lost the potential to inhibit LPS-stimulated TLR4 signaling. However, the loss of this effect was not due to C157A because MIP2-2 (C157A mutation) maintained its effect (FIG. 2D). MIP2-3 lacking the MLQALT residue at the C-terminus was also evaluated, and it demonstrated that the C-terminus was important because the effect was lost (FIG. 2D). MIP2 without CPP, CPP alone, and PFVYLI (*i.e*., a hydrophobic CPP) did not show any inhibitory effect on LPS-stimulated RAW 264.7 cells (FIG. 2E).

The regulating effects of MIP1 and MIP2 on MAL-mediated pathways were further evaluated. Although unlike MIP1, MIP2 showed a concentration-dependent inhibition of LPS-stimulated secreted alkaline phosphatase (SEAP) signal in HEK-Blue^{™} hTLR4 cells (FIG. 1A). This suggests that the hydrophobic PFV-carrier peptide shows a low cell permeability when conjugated to MIP, or has an additional TLR4-inhibitory effect when CPP is bound to MIP. Furthermore, the present inventors observed that the PFV-conjugated peptide binds to an extracellular target. CPP was tested alone so as to rule out the possibility that CPP had a regulating effect on MIP, but no TLR4 inhibitory effect was observed (data not shown). Therefore, it was confirmed that MIP2 maintains the TLR4 inhibitory effect, and that CPP is an effective carrier-peptide.

### Example 4: Confirmation of extensive TLR inhibition of MIP2, and MyD88- and TRIF-dependent inhibition of TLR4 signaling in mouse macrophages

In order to examine whether MIP2 has a broad TLR-inhibitory effect and arrests MyD88- and TRIF-dependent TLR4-signaling, further experiments were performed in RAW264.7 cells. In the absence of a cytotoxic effect at concentration of 50 µM or below (FIG. 2B), MIP2 completely removed IL-6 and significantly reduced TNF-α secretion in LPS-stimulated cells (FIGS. 1D and 1E).

In order to evaluate the effect of MIP2 on other TLRs, the level of TNF-α secretion was measured in RAW264.7 cells after stimulating by other TLR ligands (e.g., PAM₃CSK₄ (TLR1/2), FSL-1(TLR2/6), Poly(I:C) (TLR3), Resiquimod (R848; TLR7/8), Imiquimod (IMQ; TLR7), and CpG-ODN (TLR9)). All of the TLRs except TLR3 were inhibited in a concentration-dependent manner, and TLR7 showed a higher inhibitory effect than other TLRs (FIG. 1F). The inhibitory effect for TLR1/2 and TLR2/6 was relatively the same. In contrast, Couture *et al.* observed that TR6 significantly inhibited the mRNA levels of 3C (TLR1/2 ligand)-induced TNF-α and IL-1β, but not against the P2C (TLR2/6 ligand)-induced effects. Furthermore, this result suggests that MIP2 significantly inhibits endosomal TLR. As a result of Western blot, it was found that MIP2 substantially inhibits the phosphorylation of ERK and JNK (FIG. 3A). It was reported that, unlike MIP2, TR6 only inhibited ERK but not JNK in LPS-stimulated RAW264.7 cells.

TLR7 and TLR9 require TIRAP. Nevertheless, these receptors responded normally to TLR3, TLR5, TLR7 and TLR9 ligands in TIRAP-deficient cells and mice experiments, and many *in vivo* and *in vitro* studies have demonstrated that the response to TLR2 and TLR4 ligands causes an impairment of cytokine induction (Yamamoto, M. et al., Nature 420, 324-329 (2002)). MIP2 binds strongly to a wide range of TIR-containing molecules. This corresponds to the fact that TR6 (including residues overlapping with MIP2) precludes the interaction with TRAM and TLR4 of MyD88 (Piao, W., et al., J Immunol 190, 2263-2272 (2013)), and physically interacts with the TIR domain of MyD88.

While TLR4 can activate interferon only through the TRAM-TRIF pathway, NF-κB can be activated through both MyD88 and TRIF (Yamamoto, M. et al., Science 301, 640-643 (2003)). It was confirmed that MAL and TRAM later bind together to promote TRIF or MyD88 interaction with TLR4. This is because MAL and TRAM share the TLR4 interface and compete for binding. Therefore, the present inventors thought that MIP2 could inhibit both pathways of TLR4. MIP2 inhibited phosphorylation of IRF3 and expression of ATF3 in LPS-stimulated RAW264.7 cells (FIG. 3B). This effect was repeated in THP-1 cells, and MIP2 inhibited phosphorylation of IRF3 and IRF7. Since IRF3 expression and IFN production are interdependent (Honda, K. & Taniguchi, T., Nat Rev Immunol 6, 644-658 (2006)), the present inventors did not perform any additional analysis to confirm the induction of type-1 IFN.

The TLR4-inhibitory effect of MIP2 was further confirmed through the levels of expression of NF-κB and nuclear translocation. It was confirmed through Western blot data that MIP2 inhibited LPS-induced phosphorylated NF-κB (p-p65) and stopped Iκ-Bα degradation (FIG. 3C). This result was further confirmed by confocal microscopy analysis, where MIP2 completely inhibited the phosphorylation of the p65 subunit of NF-κB and its translocation into the nucleus (FIG. 3D). Stimulation of TLR is associated with induction of nitric oxide (NO) and reactive oxygen species (ROS), and expression of related enzymes (e.g., nitric oxide synthase (iNOS) and cyclooxygenase 2 (COX2)) (West, A. P. et al., Nature 472, 476-480 (2011)). MIP2 completely inhibits the expression of iNOS and COX2 (FIG. 3E), the extracellular and intracellular release of NO (FIGS. 3F and 3G, respectively), and the generation of intracellular ROS (FIG. 3H).

### Example 5: Confirmation of Inhibition of TLR and NLRP3 Pathways in THP-1 Cells of MIP2

Consistent with the results observed in RAW264.7 cells, MIP2 exhibited an inhibitory effect on MAPK in LPS-stimulated THP-1-derived macrophages. In MIP2-treated RAW264.7 cells, p38 expression was slightly suppressed, and this inhibitory effect was observed to be more distinct in LPS-stimulated THP-1 cells (FIG. 4A). Expression of p-p65 and other transcription factors (*e.g*., p-ERK and p-JNK) was substantially reduced in LPS-stimulated cells. The secretion of TNF-α and IL-6 was inhibited in LPS-stimulated cells in an MIP2 concentration-dependent manner (FIG. 4B).

It has been demonstrated that TLR upregulates the expression of NLRP3, caspase-1, and pro-IL-1β (Bauernfeind, F. G. et al., J Immunol 183, 787-791 (2009)). It has been reported that in the absence of a secondary signal (e.g., ATP, Nigericin, Streptolysin O, and Uric acid crystals), LPS-primed cells upregulate pro-IL-1β, but these cells are not treated with IL-1β and are released. In addition, for sensitization and activation of the NLRP3 inflammasome, TLR employs both the MyD88- and TRIF- pathways. This was confirmed in a Western blot experiment of the present invention in which LPS-sensitized cells expressed NLRP3 upon ATP treatment. However, both NLRP3 and pro-IL-1β expression were substantially inhibited in the presence of MIP2 (FIG. 4C).

Induction of processed IL-1β, which is released after treating pro-IL-1β with NLRP3-caspase-1, was significantly inhibited in LPS-sensitized ATP-treated cells (bottom of FIG. 4C). In addition to IRF3, IRF7 is also expressed downstream of TLR4 in a TRIF-dependent manner (Fitzgerald, K. A. et al., J Exp Med 198, 1043-1055 (2003)). LPS and CpG-ODN induce the expression of IRF3 and IRF7 and provide neuroprotection in mice with middle cerebral artery occlusion (MCAO) (Stevens, S. L. et al., J Neurosci 31, 8456-8463 (2011)). Consistent with the results observed in RAW264.7 cells, MIP2 inhibited not only the expression of IRF3 but also the expression of IRF7. In addition, the expression of pro-IL-1β and p-IRAK4 was inhibited in LPS-stimulated cells (FIG. 4D).

These results suggest that MIP2 not only affects MyD88- and TRIF-dependent cytokine production, but also inhibits TLR-mediated NLRP3 inflammasome assembly.

### Example 6: Confirmation of effect of MIP2 on imiquimod-induced psoriasis

Psoriasis is a chronic inflammatory skin disease which is characterized by amplified proliferation and altered differentiation of epidermal cells as well as invasion of inflammatory cells into the skin. TLRs are widely associated with the onset and progression of psoriasis (Hirai, T. et al., J Immunol 190, 4805-4811 (2013)).

In order to examine whether MIP2 possesses the potential for treating TLR-induced psoriasis, a disease was induced by topical treatment of 62.5 mg of 5% IMQ-cream on the back skin in 6-7 week-old C57BL/6 mice for 5 consecutive days (FIG. 5A). MIP2 was injected intraperitoneally (i.p.) for 6 days at a dose of 1 nmol/g, 10 nmol/g, or 20 nmol/g (mouse weight). The same protocol was applied to mice which were injected with methotrexate (MTX) and PBS. Through comparative phenotypic examination, it was confirmed that MIP2 attenuated disease symptoms to a degree similar to that of MTX (FIG. 5A).

In the PBS-injected mice, symptoms continued to progress and were prominent. According to the data of the Psoriasis Area and Severity Index (PASI, a tool widely used for assessing the severity of psoriasis), although no concentration-dependent effect was observed, it was confirmed that MIP2-treatment significantly reduced the PASI index (FIG. 5B).

Splenomegaly is widely associated with psoriasis (Carceller, E. et al., Sci Rep 6, 38825 (2016)). A concentration-dependent decrease in spleen weight was shown in MIP2-treated mice (FIG. 5C). The data monitoring body weight showed no significant difference compared to PBS- and MTX-treated mice (FIG. 5D). Histopathological changes in the skin were evaluated through Haemotoxylin and Eosin (H&E) staining. The thickness of the epidermis (grey arrow) and dermis (black arrow) in MIP2-treated mice was substantially reduced, almost similar to that in MTX-treated mice (FIG. 5E).

A hyperproliferative condition of the skin and infiltration of immune cells (T cells or macrophages) are the main features of psoriasis. Therefore, skin lesions from each animal group were collected and analyzed through an immunohistochemical technique using CD68 (a macrophage marker), CD4 and IL17 (TH17 cell markers) antibodies. MIP2 reduced overexpression of CD68, CD4, and IL-17 (brown staining) in psoriasis-mouse, which was similar to the MTX-treated group (FIG. 5F).

Summarizing these data, MIP2 is expected to alleviate the symptoms of induced psoriasis. This effect may be due to TLR7, which is a common target for imquimod (which induces psoriasis) and MIP2 (which inhibits the TLR7 pathway). In order to confirm this, the present inventors, the therapeutic effect of MIP2 was evaluated in non-TLR-induced systemic lupus erythematosus (SLE)-prone mice as shown in the following Example.

### Example 7: Confirmation of therapeutic effect of MIP2 in systemic lupus erythematosus (SLE) mouse model

The severity and pathogenesis of SLE, which is a disease characterized by a lack of resistance of the body to self-nuclear antigens, is broadly associated with upregulation of TLRs (Wu, Y. W., Tang, W. & Zuo, J. P., Acta Pharmacol Sin 36, 1395-1407 (2015)). Therefore, the present inventors made an attempt to evaluate the therapeutic potential of MIP2 in the SLE mouse model.

MRL lymphoproliferative mice (so-called MRL/lpr, these mice spontaneously develop characteristic serological markers and peripheral pathologies, typically lupus) were intraperitoneally injected with MIP2 at a dose of 10 nmol/g per mouse body weight for 20 days (FIG. 6A). While a significant decrease of hair loss was observed in SLE-prone MIP2-treated mice, it continued to worsen in untreated mice (FIG. 6A). A close relationship between weight gain and increased SLE markers (ANA, ROS, and splenomegaly), as well as damage to the heart, kidney, liver and spleen, has been reported (Toller-Kawahisa, J. E. et al., Free Radic Biol Med 86, 362-373 (2015)). The present inventors have confirmed that MIP2 prevents weight gain/loss in mice fed with a standard diet (FIG. 6B).

Lymphadenopathy and splenomegaly are symptoms widely observed in patients with SLE. Lymphadenopathy and splenomegaly occur in approximately 50% and 10-40% of patients with SLE, respectively (Bashal, F., Open Rheumatol J7, 87-95 (2013)). It was confirmed that the size and weight of lymph nodes were significantly reduced in MIP2-treated SLE-prone mice (FIGS. 6D, 6E, and 2G).

Characteristics of SLE-associated serological markers (*e.g*., interleukin (IL), anti-dsDNA antibody, and antinuclear antibody (ANA) and urine albumin levels (associated with glomerulonephritis)) appear in SLE patients and animal models. In addition, relatively low serum levels of complement components, particularly C3 and C4, are common markers of SLE (Sandhu, V. & Quan, M., Open Rheumatol J 12, 171 (2018)). The present inventors compared and monitored these markers in MIP2-treated and untreated MRL/lpr mice. Albumin levels were significantly reduced in MIP2-treated MRL/lpr mice (FIG. 6F). In addition, significant reductions in ANA and anti-dsDNA antibodies were observed compared to untreated mice (FIGS. 6G and 6H). A significant reduction in serum-IL6 levels was recorded, and an elevation of serum-C3 in MIP2-treated mice was confirmed (FIG. 6I and 6J).

Then, the SLE-treatment effect of MIP2 was examined by histological examination of mouse kidneys through H&E and PAS staining (FIG. 6K, scale bar 100µm). Glomerular masses with proliferative mesangial cells and diffused black broken circles were found in untreated mice. After the MIP2 treatment, fibrocellular crescent was reformed, and severe mesangial cells, proliferation of endothelial cells, and inflammatory cell invasion were substantially restored around the glomeruli. Consequently, it was confirmed that MIP2 has a significant potential in the treatment of SLE.

### Example 8: Confirmation of the therapeutic effect of MIP2 for rheumatoid arthritis (RA)

In order to expand the therapeutic potential of MIP2 in immune diseases, the effect of MIP2 in male Lewis RA rats was evaluated. The rats were divided into three groups, each group consisting of three rats of 1) normal/healthy rats; 2) untreated RA rats; 3) MIP2-treated RA rats. The rats in Groups 2 and 3 were injected with MIP2 into the knee joint, and Group 1 was injected with PBS. In this Example, rats were phenotypically evaluated for RA symptoms. Although pathological examination was not performed, the therapeutic effect of MIP2 may be reproduced in the future. The effect of MIP2 was evaluated by visually monitoring the paws of the rats, measuring the ankle diameter, and measuring the articular index (AI) for six weeks at one week intervals.

During the six weeks after the RA procedure, joint distension, severe edema, erythema, and stiffness of the ankle bones (sequester) increased significantly in untreated rats. MIP2-treated rats showed significantly reductions in edema and ankle swelling after 2-3 weeks of treatment, and recovered physiologically normal paws during the last 2 weeks of treatment regimen (FIG. 7A). The evaluations of AI index and ankle diameter confirmed that while the MIP2-treated mice became normal during 5-6 weeks of treatment, the untreated RA rats showed an increased index (FIG. 7B).

Consequently, it is suggested that MIP2 has a broad therapeutic effect on immune-related diseases and may become a promising therapeutic agent targeting autoimmune diseases in the future.

### Example 9: Confirmation of preventive and therapeutic effects of MIP2 on nonalcoholic steatohepatitis (NASH)

The pathophysiology of nonalcoholic steatohepatitis (NASH) has been implicated in several organs (*e.g*., liver, adipose tissue, gastrointestinal tract, and brain), and the TLR pathway is one of the key pathways in NASH pathogenesis. It has been reported that TLR7- or TLR4 knockout mice can be protected from non-alcoholic fatty liver disease (Rivera CA, et al., J Hepatol 2007, 47; 571-579; Roh YS, et al., The American Journal of Pathology. 188; 2574-2588).

In order to determine whether MIP2 prevents or treats NASH, NASH was induced in 10-week-old C57BL/6J mice using a methionine-choline-deficient (MCD) diet for 6 weeks. As a preventative or therapeutic strategy, MIP2 was intraperitoneally (i.p.) injected for 6 or 4 weeks at a dose of 10 nmol/g (mouse weight). Liver tissues stained with H & E, Masson-trichrome, and Oil red O staining showed a severe change in macrovesicular fat accompanied with moderate inflammation in the MCD diet group. The MIP2 prevention group or the MIP2 treatment group did not show any significant pathological improvement of steatosis (FIG. 8A). Although there was no significant overall change, the levels of hepatic cholesterol and triglycerides were significantly decreased when MIP2 was administered (FIG. 8B). Similarly, although there were no significant changes in serum alanine aminotransferase and aspartate aminotransferase in the MIP2 prevention group or the MIP2 treatment group (FIG. 8C), hepatic expression of the inflammatory transcription factors (*i.e*., TNF-α, Mcp1, IL-1β, and IL-6) was decreased by the administration of MIP2 (FIG. 8D). Consistent with the MIP2-mediated improvement in liver inflammation, the hepatic expression of the fibrotic transcription factors Col, Fn1, and Acta2 was reduced (FIG. 8E). Choline deficiency decreased Pparα and increased Pparγ transcription factor, and MIP2 restored both transcription factors in the MCD-induced NASH mouse model (FIG. 8F).

Consequently, MIP2 reduced hepatic fat content, inflammation, and the expression of fibrosis-related gene, and restored the dysfunctional PPAR pathway in the NASH mouse model induced by the MCD diet. The effect of MIP2 on NASH was more effective for prevention than treatment.

### Industrial Applicability

The peptide according to the present invention exhibits an inhibitory effect on a wide range of TLR signaling pathways including TLR4, blocks MyD88- and TRIF-dependent TLR4 pathways, and has a substantial disease-alleviating effect in mouse models with rheumatoid arthritis, psoriasis, systemic lupus erythematosus, and nonalcoholic steatohepatitis (NASH), and thus can be effectively used as a therapeutic agent for immune-related diseases and inflammatory diseases requiring negative regulation of TLR.

From the foregoing, specific parts of the present invention have been described in detail, and it will be apparent to those of ordinary skill in the art that the specific description above are mere preferred embodiments and the scope of the present invention should not be limited thereby. Accordingly, the substantial scope of the invention should be defined by the appended claims and equivalents thereof.

## Claims

1. A peptide represented by the amino acid sequence of SEQ ID NO: 1 or SEQ ID NO: 3.

2. The peptide of claim 1, wherein the peptide inhibits a signaling pathway of any one or more Toll-like receptors (TLRs) selected from the group consisting of TLR4, TLR1/2, TLR2/6, TLR3, TLR7/8, TLR7, and TLR9.

3. The peptide of claim 1, wherein the peptide is derived from an αC helix of MAL (TIR domain adaptor protein; TIRAP).

4. The peptide of claim 1, wherein the peptide binds to a Toll/interleukin-1 receptor (TIR) domain or a TIR-containing adapter molecule.

5. A fusion peptide, wherein a cell penetrating peptide is bound to the peptide of claim 1.

6. The fusion peptide of claim 5, wherein the cell penetrating peptide is represented by the amino acid sequence of SEQ ID NO: 6.

7. The fusion peptide of claim 5, wherein the fusion peptide is represented by the amino acid sequence of SEQ ID NO: 8.

8. A Toll-like receptor (TLR) antagonist comprising the peptide of claim 1 or the fusion peptide of claim 5.

9. The TLR antagonist of claim 8, wherein the TLR is any one or more selected from the group consisting of TLR4, TLR1/2, TLR2/6, TLR3, TLR7/8, TLR7, and TLR9.

10. A composition for preventing or treating autoimmune diseases comprising the peptide of claim 1 or the fusion peptide of claim 5.

11. The composition of claim 10, wherein the autoimmune disease is selected from the group consisting of psoriasis, rheumatoid arthritis, psoriatic arthritis, experimental autoimmune arthritis, asthma, Crohn's disease, multiple sclerosis, experimental autoimmune encephalomyelitis, myasthenia gravis, thyroiditis, experimental form of uveitis, Hashimoto thyroiditis, primary myxedema, thyroid poisoning, malignant anemia, autoimmune atrophic gastritis, Addison's disease, premature menopause, male infertility, childhood diabetes, Goodpasture syndrome, common pemphigus, pemphigoid, sympathetic ophthalmitis, lens uveitis, autoimmune hemolytic anemia, idiopathic leukocytopenia, primary biliary cirrhosis, chronic active hepatitis, latent cirrhosis, ulcerative colitis, Sjogren's syndrome, scleroderma, Wegener's granulomatosis, polymyositis, skin myositis, discoid lupus, and systemic lupus erythematosus.

12. A composition for preventing or treating inflammatory diseases comprising the peptide of claim 1 or the fusion peptide of claim 5.

13. The composition of claim 12, wherein the inflammatory disease is selected from the group consisting of insulin-dependent diabetes, eczema, allergy, atopic dermatitis, acne, atopic rhinitis, pneumonia, allergic dermatitis, chronic sinusitis, contact dermatitis, seborrheic dermatitis, gastritis, gout, gouty arthritis, ulcer, chronic bronchitis, ulcerative colitis, ankylosing spondylitis, sepsis, vasculitis, bursitis, lupus, rheumatoid polymyalgia, temporal arteritis, solid tumor, Alzheimer's disease, atherosclerosis, obesity, viral infection, and nonalcoholic steatohepatitis.
